(19) Europäisches Patentamt
European Patent Office
Office européen des brevets

(11) **EP 3 592 807 B1**

(12) **EUROPÄISCHE PATENTSCHRIFT**

(45) Veröffentlichungstag und Bekanntmachung des Hinweises auf die Patenterteilung:
**23.06.2021   Patentblatt 2021/25**

(51) Int Cl.:
***C08L 5/10*** (2006.01)      ***C08L 25/08*** (2006.01)

(21) Anmeldenummer: **18715886.0**

(22) Anmeldetag: **09.03.2018**

(86) Internationale Anmeldenummer:
**PCT/DE2018/100218**

(87) Internationale Veröffentlichungsnummer:
**WO 2018/162009 (13.09.2018 Gazette 2018/37)**

(54) **VERFAHREN UND MATERIAL ZUR DIFFERENZIERTEN SEQUESTRIERUNG VON STOFFEN VERSCHIEDENER STOFFGRUPPEN MIT HILFE VON SULFATIERTE ODER SULFONIERTE KOMPONENTEN ENTHALTENDEN HYDROGELEN**

METHOD AND MATERIAL FOR DIFFERENTIAL SEQUESTRATION OF SUBSTANCES FROM VARIOUS SUBSTANCE GROUPS WITH HYDROGELS CONTAINING SULFATED OR SULPHONATED COMPONENTS

PROCÉDÉ ET MATÉRIAU POUR LA SÉQUESTRATION DIFFÉRENTIELLE DE SUBSTANCES DE DIFFÉRENTS GROUPES DE SUBSTANCES AVEC DES HYDROGELS CONTENANT DES COMPOSANTS SULFATES OU SULFONÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.03.2017   DE 102017105195**

(43) Veröffentlichungstag der Anmeldung:
**15.01.2020   Patentblatt 2020/03**

(60) Teilanmeldung:
**21170125.5**

(73) Patentinhaber: **Leibniz-Institut für Polymerforschung Dresden e.V.
01069 Dresden (DE)**

(72) Erfinder:
• **SCHIRMER, Lucas**
  **01069 Dresden (DE)**
• **FREUDENBERG, Uwe**
  **01219 Dresden (DE)**
• **WERNER, Carsten**
  **01324 Dresden (DE)**
• **ATALLAH, Passant**
  **01099 Dresden (DE)**

(74) Vertreter: **Sperling, Thomas
Sperling, Fischer & Heyner
Patentanwälte
Tolkewitzer Straße 22
01277 Dresden (DE)**

(56) Entgegenhaltungen:
• **FREUDENBERG UWE ET AL: "Heparin desulfation modulates VEGF release and angiogenesis in diabetic wounds", JOURNAL OF CONTROLLED RELEASE, Bd. 220, 22. Oktober 2015 (2015-10-22), Seiten 79-88, XP029341131, ISSN: 0168-3659, DOI: 10.1016/J.JCONREL.2015.10.028**
• **SILTANEN CHRISTIAN ET AL: "Microfluidic fabrication of bioactive microgels for rapid formation and enhanced differentiation of stem cell spheroids", ACTA BIOMATERIALIA, ELSEVIER, AMSTERDAM, NL, Bd. 34, 13. Januar 2016 (2016-01-13), Seiten 125-132, XP029473735, ISSN: 1742-7061, DOI: 10.1016/J.ACTBIO.2016.01.012**
• **ARSHAD MAHMOOD ET AL: "Can thiolation render a low molecular weight polymer of just 20-kDa mucoadhesive?", DRUG DEVELOPMENT AND INDUSTRIAL PHARMACY, 2. Juli 2015 (2015-07-02), Seiten 1-8, XP055512213, US ISSN: 0363-9045, DOI: 10.3109/03639045.2015.1061538**

- DAVID G. BELAIR ET AL: "Design of growth factor sequestering biomaterials", CHEMICAL COMMUNICATIONS, Bd. 50, Nr. 99, 1. Januar 2014 (2014-01-01), Seiten 15651-15668, XP055512323, ISSN: 1359-7345, DOI: 10.1039/C4CC04317K
- ALISON B. PRATT ET AL: "Synthetic extracellular matrices for in situ tissue engineering", BIOTECHNOLOGY AND BIOENGINEERING, Bd. 86, Nr. 1, 12. Februar 2004 (2004-02-12), Seiten 27-36, XP055102381, ISSN: 0006-3592, DOI: 10.1002/bit.10897
- MIKHAIL V. TSURKAN ET AL: "Defined Polymer-Peptide Conjugates to Form Cell-Instructive starPEG-Heparin Matrices In Situ", ADVANCED MATERIALS, Bd. 25, Nr. 18, 14. Mai 2013 (2013-05-14), Seiten 2606-2610, XP055393137, DE ISSN: 0935-9648, DOI: 10.1002/adma.201300691
- MAHMOOD ARSHAD ET AL: "An in-vitro exploration of permeation enhancement by novel polysulfonate thiomers", INTERNATIONAL JOURNAL OF PHARMACEUTICS, ELSEVIER, NL, Bd. 496, Nr. 2, 21. Oktober 2015 (2015-10-21), Seiten 304-313, XP029343988, ISSN: 0378-5173, DOI: 10.1016/J.IJPHARM.2015.10.013
- NITANAN TODSAPON ET AL: "Neomycin-loaded poly(styrene sulfonic acid-co-maleic acid) (PSSA-MA)/polyvinyl alcohol (PVA) ion exchange nanofibers for wound dressing materials", INTERNATIONAL JOURNAL OF PHARMACEUTICS, ELSEVIER, NL, Bd. 448, Nr. 1, 16. März 2013 (2013-03-16), Seiten 71-78, XP028582053, ISSN: 0378-5173, DOI: 10.1016/J.IJPHARM.2013.03.011
- YOON JUN JIN ET AL: "Photo-crosslinkable and biodegradable Pluronic/ heparin hydrogels for local and sustained delivery of angiogenic growth factor", JOURNAL OF BIOMEDICAL MATERIALS RESEARCH. PA, WILEY PERIODICALS INC, HOBOKEN, NY, US, Bd. 83a, Nr. 3, 1. Dezember 2007 (2007-12-01), Seiten 597-605, XP009145695, ISSN: 1549-3296, DOI: 10.1002/JBM.A.31271 [gefunden am 2007-05-14]
- FREUDENBERG U ET AL: "A star-PEG-heparin hydrogel platform to aid cell replacement therapies for neurodegenerative diseases", BIOMATERIALS, ELSEVIER SCIENCE PUBLISHERS BV., BARKING, GB, Bd. 30, Nr. 28, 1. Oktober 2009 (2009-10-01), Seiten 5049-5060, XP026420872, ISSN: 0142-9612 [gefunden am 2009-06-27]

**Beschreibung**

Die Erfindung betrifft ein Verfahren zur differenzierten Sequestrierung von Stoffen verschiedener Stoffgruppen A und B mit Hilfe sulfatierte und/oder sulfonierte Komponenten enthaltender Hydrogele und der Abreicherung der Stoffe der Stoffgruppe A aus einem Biofluid bei gleichzeitiger differenzierter Freisetzung von Stoffen der Stoffgruppe A oder B aus dem sulfatierte und/oder sulfonierte Komponenten enthaltenden Hydrogel in das Biofluid und/oder der reduzierten Bindung der Stoffe der Stoffgruppe B im sulfatierte bzw. sulfonierte Komponenten enthaltenden Hydrogel. Die Erfindung betrifft des Weiteren Hydrogele, die basierend auf Poly(4-Styrensulfonsäure-Co-Maleinsäure) als eine Netzwerkkomponente und amin- oder thiolhaltigen Vernetzungsmolekülen als weitere Netzwerkkomponente, synthetisch erhältlich sind, charakterisiert werden können und für das oben genannte Verfahren als Material zur differenzierten Sequestrierung von Stoffen verschiedener Stoffgruppen einsetzbar sind. Die Stoffgruppen A und B sind keine chemisch definierten Stoffgruppen, sondern können je nach Hydrogeltyp unterschiedliche Stoffe enthalten, die in der entsprechenden Konstellation und Zusammensetzung zur erfindungsgemäßen differenzierten Sequestrierung innerhalb des Gesamtsystems führen. Die Stoffgruppen A und B sind somit durch ihr Verhalten im Gesamtsystem definiert.

Das Anwendungsgebiet der Erfindung liegt auf dem Gebiet der Biotechnologie und der Medizin, wo auf molekularer Ebene bestimmte Substanzen gezielt aus einem Biofluid entfernt und dazu in einem Hydrogel sequestriert werden und andererseits andere Substanzen gezielt nicht sequestriert sondern aus dem Hydrogel in das Biofluid gezielt freigesetzt werden. Somit handelt es sich im weiteren Sinne um ein Trennverfahren auf molekularer Ebene. Im weiteren Sinn liegt das Anwendungsgebiet der Erfindung im Bereich der Nutzung abgestuft negativ geladener Hydrogele für technische, biomedizinische und biologische Anwendungen, beispielsweise für die Kultivierung von Säugetierzellen oder eine antibakterielle Ausrüstung von Oberflächen.

Im Stand der Technik sind Hydrogele mit sulfatierten Komponenten, beispielsweise SternPEG-Glykosaminoklykan-Hydrogele, aus der WO 2010/060485 A1 bekannt und werden zum Einsatz in biotechnologischen Anwendungen oder als Implantat- oder Gewebsersatzmaterialien zum Einsatz in regenerativen Therapien erforscht.

Eine Kerneigenschaft dieser Materialien besteht in der zwischen Sulfatgruppen an Polymerketten einer Komponente der Hydrogele resultierenden Wechselwirkungen mit löslichen Proteinen, wie zum Beispiel den Stoffumsatz (Metabolismus), Transport, und Signalfunktionen kontrollierenden Proteinen wie Enzymen und Signalmolekülen, wobei die ersteren beispielsweise Proteasen, Lipolasen, Amylasen und zweiteren beispielsweise Hormone, Neurotransmitter, Zytokine, Wachstumsfaktoren und Chemokine umfassen.

Die für die Affinität zu diesen Proteinen entscheidenden Wechselwirkungen basieren dabei primär auf elektrostatischen Kräften zwischen den unter Anwendungsbedingungen negativ geladen Sulfat- oder Sulfonsäuregruppen der Hydrogele und positiv geladenen Aminosäureseitenketten von Proteinen.

Dieses Prinzip wird im Stand der Technik bereits breit für die Entwicklung von Hydrogelen, die sulfatierte Zucker (Glykosaminoglykane = GAGs) enthalten, zur nachhaltigen Freisetzung von Signalmolekülen, beispielsweise für die Wachstumsfaktoren VEGF (Vaskulärer endotheliarer Wachstumsfaktor) und FGF-2 (Fibroblasten-Wachstumsfaktor 2) genutzt. Zudem konnte über eine Reduzierung des Sulfatierungsgrades in den dabei zugrunde liegenden Stern-PEG-GAG-Hydrogelen die Freisetzung von FGF-2 und VEGF graduell erhöht werden. Synthetische, Sulfonsäuregruppen enthaltende Polymere, beispielsweise mit Polystyrensulfonsäure (PSS), J. Phys. Chem. B, 2007, 111 (13), pp 3391-3397, DOI: 10.1021/jp067707d9., oder 2-Acrylamido-2-methylpropansulfonsäure (U.S. Pat. Nos. 5,451,617 and 5,011,275 und U.S. Patent Application No. 2008/011412) als sulfonierte Komponente wurden genutzt, um Hydrogele beispielsweise für die Anwendung in Kontaktlinsen herzustellen. Gegenwärtig sind jedoch keine Sulfonsäuregruppen tragenden, kovalent vernetzten, wasserhaltigen Polymernetzwerke, das heißt Hydrogele, bekannt, bei denen die Carboxylgruppen von Poly(4-styrensulfonsäure-co-Maleinsäure) als funktionelle Gruppen für eine Vernetzung mit amingruppenhaltigen kurzen Vernetzern oder amingruppenhaltigen Polymeren genutzt wurden.

Nachteilig ist zudem, dass die Wechselwirkungen zwischen Hydrogelen, die sulfatierte bzw. sulfonierte Komponenten (bzw. Bausteine) enthalten, und Proteinen bisher meist nur aus Lösungen mit ein bis zwei Signalmolekülen und oft nicht unter physiologisch relevanten Bedingungen untersucht wurden. Im relevanten biologischen Kontext sind jedoch Biofluids mit einer Vielzahl an unterschiedlichen Proteinen mit unterschiedlichen Konzentrationen vorhanden, wobei die Signalmoleküle in Biofluids nur in niedrigen Spiegeln im 100 pg/ml - 2000 ng/ml-Bereich vorhanden sind währenddessen andere Proteine wie beispielsweise Albumin in hoher Konzentration von etwa 60 mg/ml vorliegt. Hierbei spielen die primär elektrostatischen Anziehungskräfte zwischen positiv geladenen Domänen von Signalmolekülen und den negativ geladenen Sulfat- beziehungsweise Sulfonatgruppen im Hydrogel eine wichtige Rolle, für deren Abschätzung oft der Isoelektrische Punkt (IEP) eines Proteins herangezogen werden kann. Neben der anhand des IEP abschätzbaren Nettoladung bei physiologischen Bedingungen spielen aber auch die Verteilung der Ladung, bspw. das Vorhandensein positiv geladener Ladungscluster, sowie sekundäre Wechselwirkungen, die durch Proteingröße und -Struktur sowie durch Ausbildung schwächerer, nichtionischer zwischenmolekularer Kräfte wie bspw. hydrophobe Wechselwirkungen, Wasserstoffbrückenbindungen oder Dipolwechselwirkungen eine Rolle, so dass es bisher nicht möglich ist, die absolute oder relative Bindung der Proteine zu den Hydrogelen vorherzusagen. Darüber hinaus treten zusätzlich Konkurrenzprozesse auf, die bisher oft nicht berücksichtigt wurden und beispielsweise bedingt sind durch Wechselwirkungen der Zielproteine mit Albumin, das für die Kontrolle des osmotischen Drucks und die Regulation von Transportprozessen verantwortlich ist, sowie durch Wechselwirkungen zwischen Albumin mit dem Hydrogel. Weiterhin sind die in der Literatur oft zur Vorhersage der molekularen Wechselwirkungen zwischen geladenen Polymeren und Proteinen herangezogenen Bindungskonstanten nur zur Beschreibung der Wechselwirkung einzelner Molekül-Protein-Komplexe in Lösung geeignet, währenddessen in den Hydrogelen ein starker Einfluss der benachbarten Polymerketten sowie der dreidimensionalen Verteilung der Ladungs- bzw. Affinitätszentren im Polymernetzwerk auf die resultierenden Wechselwirkungen und somit auf die Bindung der Proteine im Hydrogel besteht.

Dementsprechend konnte die Selektivität der Bindung zwischen sulfatierte und/oder sulfonierte Komponenten (Bausteinen) enthaltenden Hydrogelen und molekularen Komponenten komplexer Biofluids bisher nicht aufgeklärt werden. Mit den etablierten Methoden und Verfahren ist dementsprechend eine selektive Kontrolle der Spiegel von Signalmolekülen durch sulfatierte bzw. sulfonierte Komponenten (Bausteinen) enthaltende Hydrogele in anwendungsrelevanten Biofluids nur eingeschränkt möglich.

Die Aufgabe der Erfindung besteht darin ein Verfahren vorzuschlagen, welches eine gezielte Einflussnahme auf die Konzentration bestimmter Stoffe in Biofluiden ermöglicht.

Die Aufgabe wird durch ein Verfahren und ein Material mit den Merkmalen gemäß der selbstständigen Patentansprüche gelöst. Als für die Durchführung des Verfahrens geeignetes Material konnte ein vollsynthetisches Hydrogelsystem auf der Basis von Poly(4-Styrensulfonsäure-Co-Maleinsäure) als synthetische, unter physiologischen Bedingungen negativ geladene und somit für partiell positiv geladene Biomoleküle affine Komponente für die differenzierte Sequestrierung von Stoffen verschiedener Stoffgruppen eingesetzt werden. Weiterbildungen des Verfahrens und des Materials sind in den abhängigen Patentansprüchen angegeben. Vollsynthetisch bedeutet, dass für die Hydrogelbildung keine Komponenten biologischen Ursprungs benötigt werden. Daher kann die Gefahr von nachteiligen immunogenen Reaktionen ausgeschlossen werden.

Unter der Sequestrierung von Stoffen aus den Stoffgruppen A und B im Sinne der Erfindung soll die Bindung dieser Stoffe, wie beispielsweise Signalmoleküle, Faktoren oder Enzyme, an Affinitätszentren in einem Hydrogelmaterial und somit die Reduzierung der Konzentration bzw. die vollständige Entfernung dieser Stoffe aus einem direkt mit dem Hydrogel in Kontakt stehenden Biofluid verstanden werden. Das Hydrogel besteht aus Bausteinen, welche geladen oder ungeladen sind.

Als Sulfat- und/oder Sulfonatgruppen enthaltende Hydrogele werden Hydrogele mit folgenden Eigenschaften und folgender Zusammensetzung von der Erfindung umfasst: Polymernetzwerke, die durch kovalente (chemische) Vernetzung oder physikalischer Vernetzung, beispielsweise nach der WO 2014040591 A2, zwischen zwei Hydrogel-Komponenten oder Hydrogel-Bausteinen entstehen. Der erste Baustein beziehungsweise Komponente des Hydrogels ist ein unter physiologischen Bedingungen ungeladenes Molekül, welches auch als ungeladener Baustein (UGB) bezeichnet wird und vorzugsweise ein Molgewicht von 20 g/mol bis 100.000 g/mol aufweist. Die ungeladenen Moleküle sind dabei vorteilhaft aus der Klasse der Polyethylenglykole, Poly(2-oxazoline), Polyvinylpyrrolidone (PVP), Polyvinylalkohole (PVA), und/oder Polyacrylamide (PAM) oder eines kurzen bifunktionellen Vernetzermoleküls gewählt beziehungsweise abgeleitet. Weiterhin weist der UGB mindestens zwei funktionelle Gruppen, bevorzugt 4 bis 8 funktionelle Gruppen auf, die besonders vorteilhaft zur Vernetzung geeignet sind. Für die Vernetzung geeignete Funktionalitäten (am GB oder UGB) können Amin-, Thiol-, Carboxyl-, Anhydrid-, Maleimid-, Vinylsulfon-, Acrylat-, Hydroxyl-, Isocyanat-, Epoxid- und Aldehydgruppen oder Gruppen, die nichtkovalente Bindungen basierend auf elektrostatischen Kräften, hydrophobe Wechselwirkungen, Wasserstoffbrückenbindungen, Dipolwechselwirkungen ausbilden können, sein. Der zweite Baustein (Komponente) besteht aus einem (schwefelhaltigen) Sulfat- oder Sulfonatgruppen tragendem und somit unter physiologischen Bedingungen negativ geladenem Polymer (geladener Baustein, GB) (optional mit einem Molgewicht von 2.000 bis 250.000 g/mol) welches primär über die elektrostatische (ionische) Wechselwirkungen und in geringerem Maße über weniger starke Bindungen wie van der Waals Kräfte, Wasserstoffbrücken oder hydrophobe Wechselwirkungen Proteine im Hydrogelnetzwerk binden können. Die Affinitätszentren, welche die Bindung der Proteine maßgeblich bestimmen, sind hierbei erfindungsgemäß die unter physiologischen Bedingungen weitgehend negativ geladenen Sulfat- bzw. Sulfonsäuregruppen. Als sulfatierte bzw. sulfonierte Polymere kommen dabei aus natürlichen Quellen gewonnene sulfatierte Glykosaminoglykane wie Heparin und selektive desulfatierte Heparine, Chondroitinsulfat, Heparansulfat, Keratansulfat, sulfatierte Hyaluronsäure, ebenso sulfatierte Glycopolymere auf Basis von Mannose, Lactose, Dextran sowie polysulfonierte Verbindungen welche bspw. Styrensulfonsäure (SS), Vinylsulfonsäure (VS), 2-Acrylamido-2-methylpropansulfonsäure (AMPS), Aminopropansulfonsäure (APS) oder Anetholesulfonsäure (AS) als schwefelhaltige Monomere tragen (auch in Copolymeren mit Einheiten, die die zuvor genannten Vernetzungsgruppen enthalten) zum Einsatz.

Die Affinitätszentren, negativ geladene Sulfat- bzw. Sulfonat-Gruppen, sind bestimmungsgemäß nur entlang des GBs verteilt, als UGB wird dagegen ein die Proteinadsorption minimierender Baustein herangezogen. Eventuell auf dem GB vorhandene und nicht für die Vernetzungsreaktion genutzte Carboxylgruppen werden aufgrund der im Vergleich zu den signifikant stärker sauer wirkenden und daher vorzugsweise deprotonierten Sulfat- bzw. Sulfonatgruppen des GB als nicht relevant für die Bindung der Proteine im Hydrogel angesehen.

Physiologische Bedingungen sind wässrige Lösungen die in pH und Ionenstärke menschlichen Geweben mit einem physiologischen Salzgehalt entsprechen und somit auf etwa 0,9 % NaCl mit Phosphatpuffer auf pH 7,4 eingestellt sind.

Ein Biofluid nach der Erfindung ist eine wässrige Lösung mit einem variablen Salzgehalt, vorzugsweise einem physiologischen Salzgehalt, und einem Gemisch aus Proteinen.

Der Proteingehalt kann variieren und besteht zumeist aus wasserlöslichen, globulären Proteinen, beispielsweise Signalmolekülen, Enzymen oder Faktoren, in niedrigen Spiegeln im 100 pg/ml - 2000 ng/ml-Bereich und Albuminen, welche den osmotischen Druck und Transportprozesse im Körper regulieren und dafür beispielsweise in hoher Konzentration von etwa 60 mg/ml vorliegen.

Die Konzeption der Erfindung besteht darin, dass Unterschiede der Bindung von den Stoffumsatz (Metabolismus), Transport, und Signalfunktionen kontrollierenden Proteinen (bspw. Signalmoleküle und Enzyme) an sulfatierte bzw. sulfonierte Komponenten (GB) enthaltende Hydrogele einerseits durch die elektrostatische Wechselwirkungen zwischen GB und Proteinen, sowie die Größe und die Struktur der Proteine (zum Beispiel dem Vorhandensein charakteristischer Protein-Domänen) und andererseits maßgeblich durch die Netzwerkparameter der Hydrogele bestimmt wird. Als bestimmende Netzwerkparameter wurden dabei (1) die Sulfat- bzw. Sulfonatkonzentration im gequollenen Hydrogel (mmol Sulfat bzw. Sulfonat je ml) im unter physiologischen Bedingungen gequollenen Hydrogel und (2) die Anzahl Sulfat - oder Sulfonatgruppen je Wiederholeinheit des GB dividiert durch die Molmasse der Wiederholeinheiten des GB (Anzahl Gruppen / (g/mol)) als einer charakteristischen Größe für die Ladung des GB erfasst. Der Parameter (1) ist mit der Anzahl der Wechselwirkungszentren (entsprechend negativ geladenen Sulfat- bzw. Sulfonatgruppen) im dreidimensional gequollenen Hydrogelnetzwerk korreliert und wird daher maßgeblich die Bindung von Proteinen (Signalmolekülen, Enzymen) im Hydrogel bestimmen. Dieser Parameter schließt intermolekulare Wechselwirkungen (also Wechselwirkungen benachbarter Polymerketten) der GB mit Proteinen (Signalmolekülen, Enzymen) ein. Der Parameter (2) beschreibt dagegen die Dichte und Verteilung der Wechselwirkungszentren (entsprechend negativ geladenen Sulfat bzw. Sulfonatgruppen) entlang der verschiedenen GBs und wird somit maßgeblich die Wechselwirkungen der Proteine mit den jeweiligen einzelnen GBs bestimmen und schließt somit über die räumliche Modulation der Ladungszentren entlang der Polymerkette auch spezifische Wechselwirkungen, die bspw. für Protein-Glykosaminoglykan-Wechselwirkungen bekannt sind, mit ein (siehe Capila, I.; Linhardt, R. J. Angew Chem Int Ed Engl 2002, 41 (3), 391-412). Das Bindungs- und Freisetzungsverhalten der Hydrogele für Proteine (Signalmoleküle) wird erfindungsgemäß durch eine Überlagerung der resultierenden Hydrogelnetzwerkeigenschaften resultierend aus beiden Parametern (1) und (2) maßgeblich bestimmt und kann somit durch Spezifizierung dieser Parameter auch umfänglich beschrieben werden (siehe auch Ergebnisse für die Ausführungsbeispiele UGB1-GB1 01 bis UGB1-GB4 04).

Zudem sind sterische Effekte dahingehend zu beachten, dass für eine Bindung und Sequestrierung von Proteinen eine sterische Zugänglichkeit des Hydrogelnetzwerkes gewährleistet sein muss, d.h. die Größe der Proteine darf die Maschenweite der Hydrogele, beispielsweise abschätzbar aus dem Speichermodul der Hydrogele über die Gummielastizitätstheorie gemäß Material und Methoden, nicht überschreiten.

Durch die Kontaktierung von Biofluiden mit in ihrer Netzwerksstruktur und ihrem Sulfatierungs- bzw. Sulfonierungsgrad gezielt abstufbaren Hydrogelen ist die Konzentration von relevanten Signalmolekülen im Biofluid durch eine charakteristische Selektivität der Bindung von Signalmolekülen zum Hydrogel einstellbar und dementsprechend in einer biologischen oder biotechnologischen Anwendung kontrollierbar. Die biologische Anwendung kann dabei im medizinischen Kontext bei der Modulation löslicher Signalmoleküle zur Kontrolle der -Angiogenese, der Aussprossung von Blutgefäßen, der Immunantwort, unter anderem zur Heilung neurodegenerativer und Autoimmun- und Krebserkrankungen, Diabetes, bei der kutanen Wundheilung und der Knochenregeneration, zur Inhibition der Tumorproliferation sowie zur antiseptischen und antimikrobiellen Behandlung im oder am Körper erfolgen. Biotechnologische Anwendungen bestehen in der *in vitro* Zell- und Organkultur von embryonalen Stammzellen (ES-), induziert pluripotenten Stammzellen (iPS-), sowie weiteren nicht den ES und iPS zuzuordnenden Stamm- und Vorläuferzellen, primäre, von Patienten gewonnene Zellen, immortalisierten Zelllinien, sowie Herz-, Muskel-, Nieren-, Leber- und Nervengewebe sowie in der An- oder Abreicherung und somit der Trennung von Proteingemischen, insbesondere Signalmolekül- oder Enzymgemischen.

Ein weiterer Aspekt der Erfindung betrifft, wie bereits erwähnt, ein kovalent vernetztes Hydrogelmaterial zur Durchführung des oben genannten Verfahrens, basierend auf geladenen Bausteinen in Form von Poly(4-Styrensulfonsäure-co-Maleinsäure) und ungeladenen Bausteinen in Form von Amingruppen oder Thiolgruppen enthaltenden Polymeren oder Vernetzermolekülen mit mindestens zwei Amino- oder Thiolgruppen. Dabei sind die geladenen und ungeladenen Bausteine vernetzt zu einem Polymernetzwerk, welches erhältlich ist durch die Aktivierung der Carboxylgruppen der Poly(4-Styrensulfonsäure-co-Maleinsäure) mit 1-Ethyl-3-(3-Dimethylaminopropyl)-carbodiimid (EDC)/N-Hydroxysulfosuccinimid (Sulfo-NHS) und entweder eine direkte Vernetzung mit den Amingruppen enthaltenden Polymeren oder den Vernetzermolekülen mit den mindestens zwei Aminogruppen jeweils unter Amidbildung oder eine Funktionalisierung der aktivierten Carboxylgruppen mittels bifunktionellen Vernetzermolekülen, welche jeweils eine Aminogruppe und eine zu einer Michael-Typ-Addition fähige Gruppe enthalten, und die anschließende Vernetzung mit den Thiolgruppen enthaltenden Polymeren oder den Vernetzermolekülen mit den mindestens zwei Thiolgruppen jeweils über eine Michael-Typ-Addition. Die zur Michael-Typ-Addition fähige Gruppe ist vorzugsweise ausgewählt aus Maleimid-, Vinylsulfon- und Acrylatgruppen. Die Amin- oder Thiolgruppen enthaltenden Polymere als ungeladene Bausteine sind bevorzugt ausgewählt aus der Klasse der Polyethylenglykole (PEG), Poly(2-oxazoline) (POX), Polyvinylpyrrolidone (PVP), Polyvinylalkohole (PVA) und/oder Polyacrylamide (PAM). Die alternativ verwendeten Amin- oder Thiolgruppen enthaltenden kurzen Vernetzermoleküle sind vorzugsweise nichtpolymere bifunktionelle Vernetzermoleküle. Poly(4-Styrensulfonsäure-co-Maleinsäure) als geladener Baustein wird vorteilhafterweise mit variablen Molverhältnissen von 4-Styrensulfonsäure zu Maleinsäure im Bereich von 6:1 bis 1:6 und Molmassen im Bereich von 5000 bis 100.000 g/mol ausgewählt. Gemäß einer besonders bevorzugten Ausgestaltung der Erfindung werden als ungeladene Bausteine Polymere mit enzymatisch spaltbaren Peptiden für die Polymernetzwerkbildung genutzt. Diese enzymatisch spaltbaren Peptide weisen vorzugsweise entweder Lysin, mit einer Aminogruppe in der Seitenkette, oder Cystein, mit einer Thiolgruppe in der Seitenkette, als reaktive Aminosäure in der Peptidsequenz auf. Die enzymatisch spaltbaren Peptide sind vorteilhafterweise durch humane oder bakterielle Proteasen spaltbar, insbesondere Matrix-Metalloproteasen (MMPs)-responsive wie beispielsweise PQGIWGQ, IPVSLRSG oder VPMSMRGG, Cathepsin-responsive wie VPMSMRGG, Elastase-responsive wie beispielsweise AAPV oder APEEIMDRQ, Blutgerinnungsenzym-responsive wie beispielsweise Thrombin-responsive GGF-Pipecolinsäure-RYSWGCG oder GG-Cyclohexylalanin-ARSWGCG, FXa-responsive wie beispielsweise GGIEGRMGGWCG, Kalikrein-responsive wie beispielsweise CGGGPFRIGGWCG oder Bakterienproteasen-responsive wie beispielsweise Aureolysinresponsive ADVFEA oder AAEAA, Elastase-responsive AAPV oder die Protease IV-responsive Sequenz MKATKLVLGAVILGSTLLAG. Derartig aufgebaute Hydrogele erlauben autoregulative Freisetzungs- und Degradationsmechanismen. Gemäß einer weiteren Ausführungsform der Erfindung sind bioaktive und/oder antiadhäsive Moleküle mit einer Amino- oder Carboxylgruppe und/oder zellinstruktive Peptide, insbesondere ausgewählt aus KCWG-RGDSP, KCWG-EIDGIELT, KCWG-IKLLI, KGCWGGRNIAEIIKDI, KGCWGGSDPGYIGSRSDDSA, KGCWGGPQVTRGDVFTMP, KGCWGGKGGNGEPRGDTYRAY über Lysin oder Cystein in der Sequenz an den geladenen Baustein Poly(4-Styrensulfonsäure-co-Maleinsäure) oder an deren Derivate mit zur Michael-Typ-Addition fähigen Gruppen unter Ausbildung einer kovalenten Bindung an das Hydrogelnetzwerk angebunden. Die bioaktiven Moleküle können antimikrobielle Substanzen, zum Beispiel Antibiotika oder Antiseptika, oder pharmazeutische Wirkstoffe sein. Die antiadhäsiven Moleküle sind vorzugsweise Polyethylenglykole (PEG) oder Poly(2-oxazoline) (POX). Die zellinstruktiven Peptide sind bevorzugt von Struktur- und Funktionsproteinen der Extrazellulären Matrix abgeleitete Peptide, zum Beispiel von Kollagen, Laminin, Tenascin, Fibronektin und Vitronektin, abgeleitete Peptide. Gemäß einer vorteilhaften Ausgestaltung sind die bioaktiven und/oder antiadhäsiven und/oder zellinstruktiven Peptide über enzymatisch spaltbare Peptidsequenzen kovalent an die Hydrogelnetzwerke gekoppelt. Die enzymatisch spaltbaren Peptide sind, wie bereits erwähnt, vorzugsweise sensitiv für humane oder bakterielle Proteasen, beispielsweise MMPs, Cathepsine, Elastasen, Blutgerinnungsenzyme. Derart gestaltete Hydrogele erlauben autoregulative Freisetzungs- und Degradationsmechanismen auch unter Erhalt des Hydrogelnetzwerkes. Vorzugsweise weist das Hydrogelmaterial ein Speichermodul von 0,2 bis 22 kPa auf. Die Sulfonatkonzentration im gequollenen Netzwerk und die Anzahl von Sulfonatgruppen je Wiederholungseinheit (WE) dividiert durch die Molare Masse (MW) der Wiederholungseinheit in (g/mol) kann entsprechend den in der Tabelle 5.1 (siehe unten) definierten Bereichen unabhängig voneinander variiert werden.

Damit wird durch die Erfindung ein vollsynthetisches, stark hydratisiertes und über die Sulfonatgruppen von Poly(4-Styrensulfonsäure-co-Maleinsäure) als geladener Baustein (GB) Affinitätszentren für abzutrennende Stoffgruppen tragendes Hydrogel mit gezielt abstufbaren physikalischen und biochemischen Eigenschaften bereitgestellt. Die Poly(4-Styrensulfonsäure-co-Maleinsäure) wird dabei als geladener Baustein (GB) im Sinne des zuvor beschriebenen Hydrogelmaterials verwendet. Dieses Material bietet die Möglichkeit, alle wichtigen Funktionen der natürlichen Extrazellulären Matrix (EZM) modular, das heißt weitgehend unabhängig voneinander, abzubilden. Diese Funktionen sind im Einzelnen die Gerüst-, Stütz- und Schutzfunktion für einwachsende Zellen als erste Funktion, die Kontrolle der Zelladhäsion als zweite Funktion, die abgestufte Sequestrierung und reversible Freisetzung therapeutisch relevanter Signalmoleküle als dritte Funktion sowie die Möglichkeit des bedarfsgerechten Umbaus durch einwachsende Zellen als vierte Funktion. Die physikalischen Eigenschaften, wie Steifigkeit und Hydratisierung des Materials, sind über einen weiten Bereich einstellbar. Zu diesem Zweck wurden gemäß einer bevorzugten Ausführungsform der Erfindung kovalent vernetzte Hydrogele durch Reaktion von mit 1-Ethyl-3-(3-Dimethylaminopropyl)-carbodiimid (EDC)/N-Hydroxysulfosuccinimid (Sulfo-NHS) aktivierten Carboxylgruppen der Poly(4-Styrensulfonsäure-co-Maleinsäure) und endständigen Aminogruppen eines linearen oder sternförmig verzweigten Polyethylenglykols als ungeladener Baustein über stabile Amidgruppen dargestellt. In einer alternativen Ausführungsform, die bei der Einbettung von lebenden Zellen bevorzugt wird, wurden in einem ersten Schritt die mit 1-Ethyl-3-(3-Dimethylaminopropyl) carbodiimid/N-hydroxysulfosuccinimid (EDC/sulfo-NHS) aktivierte Carboxylgruppen Poly(4-Styrensulfonsäure-co-Maleinsäure) mittels des kurzen bifunktionellen Vernetzermoleküls N-(2-Aminoethyl)maleimid abgestuft, das heißt mit 6 bis 10 Molekülen N-(2-Aminoethyl)maleimid pro Molekül Poly(4-Styrensulfonsäure-co-Maleinsäure), funktionalisiert und anschließend aufgereinigt und isoliert. Diese Derivate der Poly(4-Styrensulfonssäure-co-Maleinsäure) mit N-(2-Aminoethyl)maleimid erlauben dann die spontane, bioorthogonale Reaktion beim Vermischen in wässrigen Lösungen oder im komplexen Biofluid mit lebenden Zellen und Biomolekülen ohne deren funktionelle Veränderung über eine Michael-Typ-Addition zwischen den Maleimidgruppen der funktionalisierten Poly(4-Styrensulfonssäure-co-Maleinsäure)-Derivate und den Thiolgruppen der thiolgruppehaltigen bifunktionellen Vernetzermoleküle oder thiolgruppenhaltigen Polymere als ungeladene Bausteine im Sinne des zuvor beschriebenen Hydrogelaufbaus. Diese Bausteine können beispielsweise enzymatisch-spaltbare Peptide sein, welche in der Sequenz die Aminosäure Cystein (die eine Thiolgruppe in der Seitenkette trägt) aufweisen, und welche zuvor an einen oder mehrere Arme eines vierarmigen Polyethylenglykols (PEG) konjugiert wurden, wie bei Tsurkan et al. 2013 (Adv. Mater. 2013, 25, 2606-2610) beschrieben. Der Vorteil dieser Art von Vernetzung besteht darin, dass aufgrund der schnellen Reaktion der Maleimide mit den freien Thiolgruppen eine gerichtete Reaktion ohne ungewollte Nebenreaktionen mit anderen Biomolekülen oder mit Proteinen der Zelloberflächen erfolgt, weshalb die Michael-Typ-Addition als bioorthogonale Vernetzungsreaktion zur Einpolymerisation lebender Zellen genutzt werden kann. Die Hydrogele können zudem über die Maleimidgruppen der Poly(4-Styrensulfonsäure-co-Maleinsäure)-Derivate mittels zellinstruktiven Peptiden, beispielsweise der Aminosäuresequenz CWGRGDSP, funktionalisiert werden. Außerdem sind die Hydrogelmaterialien mit antimikrobiell wirksamen Substanzen, beispielsweise mit positiv geladenen Antibiotika funktionalisierbar.

Alternativ zum kovalent vernetzten Hydrogelmaterial ist zur Durchführung des oben genannten Verfahrens die Anwendung eines physikalisch vernetzten Hydrogelmaterials möglich. Dieses ebenfalls vollsynthetische Material basiert auf physikalischen Wechselwirkungen zwischen geladenen Bausteinen in Form von Poly(4-Styrensulfonsäure-co-Maleinsäure) und ungeladenen Bausteinen in Form von Polymeren, wobei an die Polymere stark positiv geladene Peptidsequenzen konjugiert sind. Die stark positiv geladenen Peptidsequenzen umfassen vorzugsweise mindestens zehn Wiederholungen von Lysin oder Arginin oder mindestens fünf Wiederholungen von Dipeptidmotiven mit Lysin und Alanin oder Arginin und Alanin.

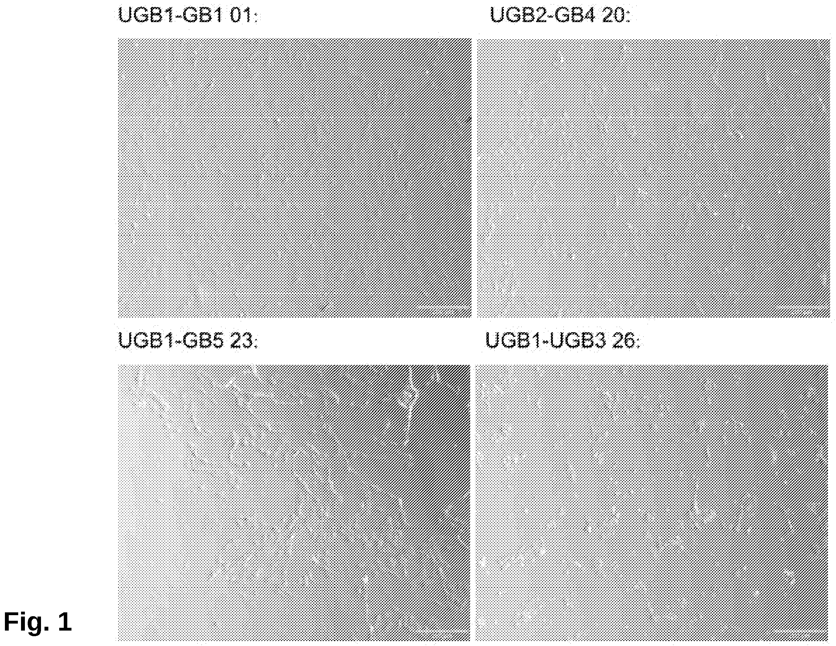

Weitere Einzelheiten, Merkmale und Vorteile von Ausgestaltungen der Erfindung ergeben sich aus der nachfolgenden Beschreibung von Ausführungsbeispielen, welche auch durch die angegebenen Tabellen und Figuren beschrieben und untersetzt werden. Die Fig. 1 zeigt repräsentative lichtmikroskopische Bilder der Zellkulturen mit humanen Endothelzellen, kultiviert auf Hydrogelen, nach 24 Stunden.

Durch die gezielte Variation der Netzwerkeigenschaften können auch andere vorteilhafte Konzentrationsverhältnisse eingestellt werden und mit einer Erweiterung der Analyse für weitere Signalmoleküle ist mit dem vorgeschlagenen Verfahren auch die Kontrolle der Konzentration dieser Spezies in beliebigen Biofluids möglich.

Tabelle 1: Chemisch-Physikalische Beschreibung der Hydrogele, Ausführungsbeispiele 01 bis 26: Vernetzungsreaktion 1 (VN1): aktivierte Carboxylgruppe mit Aminogruppe, Vernetzungsreaktion 2 (VN2): Maleimidgruppe mit Thiolgruppe

| Name | Hydrogelmischung bei der Vernetzung, ungequollen | | | Hydrogeleigenschaften unter physiologischen Bedingungen. gequollen | | | | | | | VN |
| | Konzentration UGB in mmol/ml | Konzentration GB in mmol/ml | molares Verhältnis UGB:GB | Quellung | | Konzentration GB in mmol/ml | Konzentration Sulfat/ Sulfonat in mmol/ml | Speichermodul in kPa | | |
| | | | | Mittelwert | SD | | | Mittelwert | SD | |
|---|---|---|---|---|---|---|---|---|---|---|
| UGB1-GB1 01 | 0,0091 | 0,0030 | 3 | 1,77 | 1,70 | 0,0018 | 0,12 | 1,9 | 1,9 | 1 |
| UGB1-GB2 02 | 0,0079 | 0,0040 | 2 | 1,62 | 0,03 | 0,0025 | 0,12 | 4,0 | 1,1 | 1 |
| UGB1-GB3 03 | 0,0073 | 0,0045 | 1,5 | 1,71 | 0,04 | 0,0027 | 0,06 | 2,3 | 0,0 | 1 |
| UGB1-GB4 04 | 0,0057 | 0,0038 | 1,5 | 1,23 | 0,01 | 0,0031 | 0,28 | 7,9 | 1,9 | 1 |
| UGB1-GB4 05 | 0,0079 | 0,0026 | 3 | 1,54 | 0,54 | 0,0017 | 0,15 | 14,5 | 1,6 | 1 |
| UGB1-GB4 06 | 0,0030 | 0,0020 | 1,5 | 1,54 | 0,05 | 0,0013 | 0,12 | 7,3 | 0,2 | 1 |
| UGB1-GB4 07 | 0,0041 | 0,0014 | 3 | 1,12 | 0,03 | 0,0012 | 0,11 | 13,7 | 0,5 | 1 |
| UG81-GB4 08 | 0,0052 | 0,0009 | 6 | 0,95 | 0,03 | 0,0009 | 0,08 | 19,6 | 2,0 | 1 |
| UGB1-GB4 09 | 0,0021 | 0,0014 | 1,5 | 1,25 | 0,18 | 0,0011 | 0,10 | 4,8 | 0,6 | 1 |
| UGB1-GB4 10 | 0,0030 | 0,0010 | 3 | 1,01 | 0,12 | 0,0010 | 0,09 | 8,1 | 0,7 | 1 |
| UGB1-GB4 11 | 0,0037 | 0,0006 | 6 | 0,86 | 0,11 | 0,0007 | 0,06 | 8,5 | 0,4 | 1 |
| UGB1-GB5 12 | 0,0057 | 0,0038 | 1,5 | 1,68 | 0,035 | 0,0023 | 0,09 | 8,5 | 1,7 | 1 |
| UGB1-GB5 13 | 0,0079 | 0,0026 | 3 | 1,48 | 0,23 | 0,0018 | 0,13 | 15,3 | 0,7 | 1 |

(fortgesetzt)

| Name | Hydrogelmischung bei der Vernetzung, ungequollen | | | Hydrogeleigenschaften unter physiologischen Bedingungen, gequollen | | | | | | | VN |
| | Konzentration UGB in mmol/ml | Konzentration GB in mmol/ml | molares Verhältnis UGB:GB | Quellung | | Konzentration GB in mmol/ml | Konzentration Sulfat/ Sulfonat in mmol/ml | Speichermodul in kPa | | |
| | | | | Mittelwert | SD | | | Mittelwert | SD | |
| --- | --- | --- | --- | --- | --- | --- | --- | --- | --- | --- |
| UGB1-GB5 14 | 0,0030 | 0,0020 | 1,5 | 1,81 | 0,04 | 0,0011 | 0,08 | 5,5 | 1,4 | 1 |
| UGB1-GB5 15 | 0,0041 | 0,0014 | 3 | 1,28 | 0,08 | 0,0011 | 0,08 | 12,5 | 1,2 | 1 |
| UGB1-GB5 16 | 0,0052 | 0,0009 | 6 | 0,99 | 0,04 | 0,0009 | 0,06 | 21,8 | 0,8 | 1 |
| UGB1-GB5 17 | 0,0021 | 0,0014 | 1,5 | 1,58 | 0,08 | 0,0009 | 0,07 | 3,0 | 0,2 | 1 |
| UGB1-GB5 18 | 0,0030 | 0,0010 | 3 | 1,05 | 0,10 | 0,0009 | 0,07 | 6,9 | 0,7 | 1 |
| UGB1-GB5 19 | 0,0037 | 0,0006 | 6 | 0,90 | 0,04 | 0,0007 | 0,05 | 8,0 | 1,3 | 1 |
| UGB2-GB4 20 | 0,0015 | 0,0022 | 1,5 | 1,22 | 0,03 | 0,0018 | 0,16 | 3,7 | 0,5 | 2 |
| UG82-GB4 21 | 0,0015 | 0,0015 | 1 | 1,62 | 0,20 | 0,0009 | 0,08 | 1,1 | 0,2 | 2 |
| UGB2-GB4 22 | 0,0015 | 0,0011 | 0,75 | 2,20 | 0,19 | 0,0005 | 0,05 | 0,2 | 0,1 | 2 |
| UGB2-GB5 23 | 0,0015 | 0,0022 | 1,5 | 1,19 | 0,17 | 0,0018 | 0,14 | 4,6 | 1,5 | 2 |
| UGB2-GB5 24 | 0,0015 | 0,0015 | 1 | 1,56 | 0,07 | 0,0010 | 0,07 | 1,2 | 0,2 | 2 |
| UGB2-GB5 25 | 0,0015 | 0,0011 | 0,75 | 1,95 | 0,72 | 0,0006 | 0,04 | 0,3 | 0,0 | 2 |
| UGB2-UGB3 26 | 0,0018 | 0,0000 | 1 | 1,26 | 0,25 | 0,0000 | 0,00 | 4,4 | 0,5 | 2 |

Tabelle 2: Chemisch-Physikalische Eigenschaften der polymeren Hydrogelbausteine (Komponenten); MWP: Molmasse des Polymers, MWWE: Molmasse der Wiederholeinheit:

| Polymer | Abbkürzung | MWP in g/mol | MWWE in g/mol | Anzahl Sulfat/ Sulfonat je WE | Anzahl Sulfat/ Sulfonat je Polymermaleküt |
|---|---|---|---|---|---|
| Heparin (HEP) | GB1 | 14.000 | 540 | 2,7 | 70,2 |
| N-desulfattertes Heparin (N-DSH) | GB2 | 13,600 | 523 | 1,8 | 48,4 |
| SO, N-desulfatietes Heparin (ON-DSH) | GB3 | 12,320 | 474 | 0,9 | 23,4 |
| Poly{4-Styrensulfonsäure-co-Maleinsäure), mol. Verhältnis Sulfonsäure zu Maleinsaüre: 3:1 | GB4 | 20.000 | 660 | 3,0 | 90,0 |
| Poly(4-Styrensulfonsäure-co-Maleinsäure), mol. Verhältnis Sulfonsäure zu Maleinsäure: 1:1 | GB5 | 20.000 | 265 | 1,0 | 75,0 |
| 4-Arm-PEG. aminterminiert | UGB1 | 10.000 | 44 | 0,0 | 0,0 |
| 4-Arm PEG thiolterminiert | UGB2 | 10.000 | 44 | 0,0 | 0,0 |
| 4-Arm PEG maleimidterminiert | UGB3 | 10.000 | 44 | 0,0 | 0,0 |
| 4-Arm PEG, terminiert mit einer enzymatisch spaltbaren Peptidsequenz | UGS4 | 15.600 | 44 | 0 | 0 |

Tabelle 3: Chemisch-Physikalische Eigenschaften der Signalmoleküle, Bindung bzw. Sequestrierung der Proteine im Hydrogel der Typen UGB1-GB1 01, UGB1-GB2 02, UGB1-GB5 23, UGB1-GB3 03, UGB1-GB4 04, UGB2-GB4 20 und dem ungeladenen Hydrogel UGB2-UGB3 26 (als Negativkontrolle)

Bindung der Proteine in %, vom Hydrogel mit dem Namen

| | Name | physikalisch-chemische Eigenschaften | | | | strukturelle Eigenschaften | UGB1-GB1 01 | | UGB1-GB2 02 | | UGB2-GB5 23 | | UGB1-GB3 03 | | UGB1-GB4 04 | | UGB2-GB4 20 | | UGB2-UGB3 26 | |
|---|---|---|---|---|---|---|---|---|---|---|---|---|---|---|---|---|---|---|---|---|
| | | Uniprot ID | Molekulargewicht in Dalton | IEP | Anzahl pos. gelad. Amino- | PROSITE-Sequenz Familien | Mittelwert | SD | Mittelwert | SD | Mittelwert | SD | Mittelwert | SD | Mittelwert | SD | Mittelwert | SD | Mittelwert | SD |
| Wachstumsfaktoren | FGF-2 | P09038 | 16407.6 | 9.6 | 28 | PS00247 | 31.0% | 3.2% | 5.4% | 8.7% | | | 0.0% | 19.7% | | | | | | |
| | TGFb1 | P01137 | 12794.7 | 8.6 | 15 | PS00250 | 18.0% | 4.2% | 0.0% | 2.0% | | | 0.0% | 5.9% | | | | | | |
| | bNGF | P01138 | 13494.2 | 9.0 | 21 | PS00248 | 83.5% | 7.3% | 78.5% | 4.5% | 86.8% | 0.9% | 71.4% | 3.8% | 97.3% | 2.2% | 100.0% | 0.0% | | |
| | EGF | P01133 | 6222.0 | 4.6 | 7 | PS50026 | 7.2% | 5.1% | 8.1% | 3.0% | 11.6% | 9.8% | 11.0% | 0.9% | 28.3% | 3.0% | 36.6% | 3.4% | | |
| | HGF | P14210 | 53683.7 | 7.7 | 78 | PS50070 | 41.7% | 4.0% | 49.3% | 5.2% | 49.6% | 14.6% | 41.3% | 1.5% | 41.3% | 11.8% | 80.2% | 4.0% | 19.7% | 23.1% |
| | PDGF-BB | P01127 | 12294.4 | 9.4 | 19 | PS50278 | 100.0% | 10.1% | 100.0% | 6.5% | 100.0% | 8.5% | 100.0% | 2.2% | 100.0% | 5.5% | 100.0% | 2.0% | | |
| | PLGF | P49763 | 22785.9 | 8.4 | 31 | PS50279 | 2.3% | 3.3% | 6.9% | 5.3% | 82.9% | 8.5% | 7.7% | 1.1% | 72.0% | 7.8% | 97.0% | 1.3% | | |
| | VEGF-A | P15692 | 23894.4 | 9.2 | 47 | PS50280 | 90.0% | 1.1% | 85.6% | 1.1% | 96.3% | 2.3% | 93.4% | 4.5% | 99.5% | 1.3% | 97.2% | 1.6% | 1.9% | 4.7% |
| Chemokine | Eotaxin | P51671 | 6364.8 | 9.9 | 16 | PS00472 | 100.0% | 0.0% | 98.0% | 0.4% | 98.4% | 1.0% | 91.9% | 2.3% | 89.1% | 1.3% | 96.8% | 2.6% | <0.1% | 5.1% |
| | GRO-alpha | P09341 | 7865.1 | 9.5 | 12 | PS00472 | 98.4% | 0.5% | 79.3% | 0.9% | 96.8% | 2.1% | 57.9% | 2.7% | 98.8% | 0.5% | 98.2% | 1.3% | <0.1% | 8.5% |
| | IL-8 | P10145 | 8922.3 | 9.2 | 17 | PS00471 | 94.2% | 1.3% | 89.6% | 2.1% | 94.8% | 7.3% | 44.7% | 2.5% | 98.7% | 0.8% | 95.4% | 1.3% | <0.1% | 1.0% |
| | IP-10 | P02778 | 8646.2 | 10.2 | 17 | PS00471 | 98.2% | 0.5% | 92.7% | 2.3% | 94.7% | 4.1% | 85.5% | 7.7% | 97.4% | 0.7% | 59.6% | 1.5% | 16.1% | 16.4% |
| | MCP-1 | P13500 | 9884.9 | 9.4 | 14 | PS00472 | 94.6% | 1.6% | 88.4% | 0.7% | 99.8% | 1.8% | 45.3% | 4.3% | 94.8% | 1.4% | 72.0% | 4.5% | <0.1% | 1.7% |
| | MIP-1 alpha | P10147 | 7716.5 | 4.8 | 8 | PS00472 | 58.2% | 2.7% | 41.1% | 1.2% | 81.8% | 12.1% | 20.7% | 3.7% | 94.8% | 2.9% | 94.8% | 1.4% | <0.1% | 1.7% |
| | MIP-1 beta | P13236 | 7818.7 | 4.8 | 6 | PS00472 | 56.1% | 2.2% | 38.0% | 2.7% | 78.0% | 15.7% | 23.3% | 3.1% | 94.0% | 4.1% | 97.0% | 1.3% | 0.2% | 1.9% |
| | RANTES | P13501 | 7563.6 | 9.3 | 11 | PS00472 | 99.0% | 0.2% | 95.2% | 0.6% | 94.7% | 2.4% | 90.2% | 0.5% | 99.5% | 0.7% | 94.0% | 1.3% | <0.1% | 1.5% |
| | SDF-1 alpha | P48061 | 6526.1 | 10.1 | 17 | PS00471 | 98.6% | 0.3% | 99.5% | 0.6% | 99.9% | 1.9% | 80.6% | 1.5% | 99.7% | 0.2% | 97.2% | 1.6% | <0.1% | 2.9% |
| Zytokine | GM-CSF | P04141 | 14477.4 | 5.2 | 15 | PS00702 | 6.4% | 2.1% | 3.9% | 1.0% | 7.5% | 14.1% | 11.1% | 2.5% | 24.2% | 1.7% | 36.0% | 4.5% | 1.3% | 4.2% |
| | IFN-gamma | P01579 | 16177.3 | 9.5 | 28 | PS00471 | 98.5% | 0.1% | 92.6% | 2.0% | 75.8% | 14.1% | 75.8% | 14.0% | 89.0% | 0.9% | 89.0% | 0.9% | | |
| | IL-1 beta | P01584 | 17376.7 | 5.9 | 19 | PS00253 | 27.0% | 3.7% | 25.2% | 0.6% | 25.2% | 8.1% | 26.2% | 3.6% | 26.2% | 1.9% | 72.0% | 1.9% | <0.1% | 5.4% |
| | IL-10 | P22301 | 18647.2 | 7.6 | 25 | PS00520 | 32.7% | 9.7% | 14.7% | 5.1% | 5.5% | 11.8% | 15.5% | 5.0% | 15.5% | 0.9% | 95.4% | 4.5% | <0.1% | 2.0% |
| | IL-12p40 | P29460 | 34696.6 | 5.4 | 43 | PS00838 | 59.9% | 5.1% | 85.1% | 1.8% | 15.7% | 16.4% | 45.8% | 1.3% | 92.5% | 1.3% | 92.5% | 5.0% | <0.1% | 16.4% |
| | IL-4 | P05112 | 14963.0 | 9.3 | 26 | PS00838 | 98.5% | 0.3% | 82.4% | 2.0% | 95.9% | 7.3% | 72.6% | 0.7% | 99.1% | 0.5% | 96.8% | 2.6% | <0.1% | 2.0% |
| | IL-6 | P05231 | 20812.5 | 6.2 | 25 | PS00254 | 24.3% | 4.6% | 7.9% | 4.9% | 49.4% | 26.7% | 17.3% | 7.8% | 89.5% | 1.9% | 98.2% | 4.6% | <0.1% | 1.0% |
| | TNF-alpha | P01375 | 17352.5 | 7.0 | 19 | PS00251 | 38.9% | 3.5% | 24.7% | 3.3% | 24.7% | 3.3% | 24.8% | 3.7% | 89.2% | 1.9% | 97.6% | 1.1% | | |
| | Sclerostin | Q9BQB4 | 21522.1 | 9.6 | 38 | | 85.0% | 3.1% | 42.0% | 11.2% | | | 0.0% | 8.6% | | | | | | |
| | DKK1 | Q94907 | 25777.6 | 8.7 | 42 | | 84.0% | 6.2% | 78.0% | 3.2% | | | 36.0% | 10.1% | | | | | | |
| | gemäß Tabelle 5.1 als TYP kassifiziert | | | | | | TYP1 | | TYP2 | | TYP2 | | TYP3 | | TYP4 | | TYP4 | | TYP5 | |

11

Tabelle 4: Aktivierungszeit der Carboxylgruppen der GB 1 bis 5 in min, genutztes Gelvolumen für die Bindungsstudien

|  | Aktivierungszeit | Gelvolumen für Bindungsstudien |
|---|---|---|
| UGB1-GB1 01 | 15 min | 12.14 μl |
| UGB1-GB2 02 | 10 min | 12.38 μl |
| UGB1-GB3 03 | 45 min | 8.56 μl |
| UGB1-GB 5-19 | 1 min | 12.00 μl |
| UGB2-GB4 20 bis UGB2-UGB3 26 | 0 min | 10.00 μl |

Tabelle 5.1 Geltypen mit Parameterbereichen

|  | A | B | C | D |
|---|---|---|---|---|
| Typ 1 | 0,0040-0,0060 | 0,09-0,20 | <20 | ungeladen |
| Typ 2 | 0,0025-0,0040 | 0,05-0,18 | <20 | ungeladen |
| Typ 3 | 0,0005-0,0025 | 0,01-0,12 | <20 | ungeladen |
| Typ 4 | 0,0040-0,0100 | 0,16-0,80 | <20 | ungeladen |
| Typ 5 | 0 | 0 | <20 | ungeladen |

Tabelle 5.2 Geltypen bevorzugter Ausführungsbeispielen

|  | A | B | C | D |
|---|---|---|---|---|
| Typ 1 (UGB1-GB1 01) | 0,0050 | 0,12 | <20 | ungeladen |
| Typ 2 (UGB1-GB2 02) | 0,0035 | 0,12 | <20 | ungeladen |
| Typ 2 (UGB2-GB5 23) | 0,0038 | 0,14 | <20 | ungeladen |
| Typ 3 (UGB1-GB3 03) | 0,0019 | 0,06 | <20 | ungeladen |
| Typ 4 (UGB1-GB4 04) | 0,0045 | 0,28 | <20 | ungeladen |
| Typ 4 (UGB2-GB4 20) | 0,0045 | 0,16 | <20 | ungeladen |
| Typ 5 (UGB2-UGB3 26) | 0 | 0 | <20 | ungeladen |

Tabelle 6.1

|  | E | F | G | C | D |
|---|---|---|---|---|---|
| Typ 1 | 60-80 | 0,0015-0,0025 | 0,09-0,20 | <20 | ungeladen |
| Typ 2 | 30-75 | 0,0015-0,0030 | 0,05-0,18 | <20 | ungeladen |
| Typ 3 | 10-30 | 0,0010-0,0040 | 0,01-0,12 | <20 | ungeladen |
| Typ 4 | 80-120 | 0,0018-0,0050 | 0,16-0,80 | <20 | ungeladen |
| Typ 5 | 0 | 0 | 0 | <20 | ungeladen |

Tabelle 6.2.

|  | E | F | G | C | D |
|---|---|---|---|---|---|
| Typ 1 (UGB1-GB1 01) | 70,2 | 0,0018 | 0,12 | <20 | ungeladen |
| Typ 2 (UGB1-GB2 02) | 48,4 | 0,0025 | 0,12 | <20 | ungeladen |

(fortgesetzt)

|  | E | F | G | C | D |
|---|---|---|---|---|---|
| Typ 2 (UGB2-GB5 23) | 75,0 | 0,0018 | 0,14 | <20 | ungeladen |
| Typ 3 (UGB1-GB3 03) | 23,4 | 0,0027 | 0,06 | <20 | ungeladen |
| Typ 4 (UGB1-GB4 04) | 90,0 | 0,0031 | 0,28 | <20 | ungeladen |
| Typ 4 (UGB2-GB4 20) | 90,0 | 0,0018 | 0,16 | <20 | ungeladen |
| Typ 5 (UGB2-UGB3 26) | 0 | 0 | 0 | <20 | ungeladen |

In den Tabellen 5.1, 5.2, 6.1 und 6.2 sind in Spalte A als Eigenschaft der geladenen Bausteine die Anzahl der Sulfat- oder Sulfonatgruppen je Wiederholeinheit (WE) dividiert durch die Molare Masse (MW) der WE in [mol/g] angegeben. Als Eigenschaften der gequollenen Hydrogele sind in Spalte B die Konzentration der Sulfat- oder Sulfonatgruppen in mmol/ml und in Spalte C das Speichermodul in kPa angegeben. Spalte D gibt die Eigenschaften der ungeladenen Bausteine an.

Spalte E gibt die molare Konzentration der Sulfat- oder Sulfonatgruppen je mol Polymer (in mol/mol) des geladenen Baustein an. Die Eigenschaften der Hydrogele zeigen Spalte F als Konzentration der GB in mmol/ml, Spalte G als Konzentration der Sulfat- oder Sulfonatgruppen in mmol/ml und Spalte C als Speichermodul.

Das Speichermodul von 20 kPa entspricht nach der in den Methoden aufgeführten Annahmen in etwa einer Maschenweite von 6 nm und sollte somit eine sterische Zugänglichkeit aller hier diskutierten und der strukturell ähnlichen Signalmoleküle in die Hydrogele ermöglichen.

Tabelle 7

| Name | Konzentration / pg/ml |
|---|---|
| FGF-2 | 550 |
| TGFbl | 4047 |
| bNGF | 29500 |
| EGF | 11986 |
| HGF | 30600 |
| PDGF-BB | 26000 |
| PLGF | 9971 |
| VEGF-A | 48290 |
| Eotaxin | 2500 |
| GRO-alpha | 9500 |
| IL-8 | 12386 |
| IP-10 | 8800 |
| MCP-1 | 6500 |
| MIP-1 alpha | 6400 |
| MIP-1 beta | 13500 |

| | |
|---|---|
| RANTES | 3600 |
| SDF-1 alpha | 39800 |
| GM-CSF | 41000 |
| IFN-gamma | 50700 |
| IL-1 beta | 8250 |

(fortgesetzt)

| IL-10 | 9250 |
|---|---|
| IL-12p40 | 13500 |
| IL-4 | 36200 |
| IL-6 | 44368 |
| TNF -alpha | 31363 |
| Sclerostin | 13300 |
| DKK1 | 864 |

In Tabelle 7 sind Stoffe der Stoffgruppen A und B in relevanten Konzentrationen in den Biofluiden aufgeführt.

Die Erfindung wird nachfolgend mit Bezugnahme der Ausführungsbeispiele (AB) auf die vorangehend gezeigten Tabellen erläutert.

Beispielsweise erweisen sich Hydrogele aus UGB1-GB1 01 gemäß Tabelle 1 (AB1) als besonders vorteilhaft in der Einstellung der Konzentrationen von verschiedenen Signalmolekülen (siehe Tabelle 3). Die Klasse der Chemokine weist eine hohe strukturelle Ähnlichkeit der Tertiärstruktur auf, welche durch die Interaktion von vier Cysteinen durch Disulfidbrücken stabilisiert wird (entsprechend PROSITE ID: PS00471 und PS00472). Des weiteren sind Chemokine durch eine stark positive Nettoladung IEP >9 oder positiv geladenen Domänen wie bei MIP1-alpha und MIP1-beta und einem Molekulargewicht von unter 10 kDa (siehe Tabelle 3) charakterisiert und werden *von diesem Hydrogeltyp* stark im Hydrogel gebunden und somit aus angrenzenden Biofluids abgereichert (Bindung von Mipl-alpha und Mip1-beta ca. 60%, die anderen Chemokine Eotaxin, GRO-alpha, IL-8, IP-10, MCP-1, Rantes und SDF-1 alpha werden ≥95% gebunden (Tabelle 3), währenddessen überraschenderweise folgende stark positiv geladene, der Klasse der Wachstumsfaktoren zuzuordnende Signalmoleküle FGF-2 (IEP 9,58) und TGFbl (IEP 8,59) sowie strukturell ähnliche Proteine der FGF Familie (entsprechend PROSITE ID: PS00247) und der TGFb1 Familie (entsprechend PROSITE ID: PS00250) mit 31% und 18% nur sehr gering, PLGF (IEP 8,37) mit einer Bindung <10% noch geringer (Tabelle 3), und die der Klasse der Zytokine zuzuordnenden Signalmoleküle mit leicht basischem IEP wie IL-10 (IEP 7,65) sowie Signalmoleküle mit einem IEP im Neutralbereich (pH 5,5-7) wie IL1-beta, IL6, und TNF-alpha und strukturell ähnliche Proteine der IL-1-beta Protein Familie (entsprechend PROSITE ID: PS00253), IL-10 Protein Familie (entsprechend PROSITE ID: PS00520), Interleukin-6 / GM-CSF / MGF Familie (entsprechend PROSITE ID: PS00254) und TNF-alpha Protein Familie (entsprechend PROSITE ID: PS00251) aus dem komplexen Biofluid nur gering ≤30% (Tabelle 3) gebunden werden, (Bindung von TNF-alpha ca. 38%) und somit mit nahezu unveränderten Konzentrationen im Biofluid vorliegen. Weiterhin wird der Wachstumsfaktor mit leicht negativer Nettoladung EGF (IEP 4,8) mit einer Bindung kleiner 10% nahezu nicht gebunden. Damit bleiben für das Überleben von Zellen essentielle Signalfaktoren wie EGF, FGF-2, TGFß oder PLGF durch die mittels der o.g. Hydrogele realisierte Sequestrierung unbeeinflusst.

Die stark basischen Zytokine IFN-gamma (IEP 9,52), IL-4 (IEP 9,25), die WNT- und BMP-Antagonisten Sclerostin (9,57) und DKK1 (8,72) sowie strukturell ähnliche Proteine der IL-4/IL-13 Familie (entsprechend PROSITE ID: PS00838) und der Sclerostin-ähnlichen Familie (entsprechend InterPro ID: IPR008835) und die stark basischen Wachstumsfaktoren bNGF (IEP 9,00), PDGF (IEP 9,4), und VEGF (IEP 9,2) sowie strukturell ähnliche Proteine der NGF Familie (entsprechend PROSITE ID: PS00248), und der PDGF Familie (entsprechend PROSITE ID: PS00250) sowie IL-12p40 werden mit ≥ 59,9% erwartungsgemäß stark gebunden (Tabelle 3) und daher aus dem Biofluid abgereichert.

Durch die Kontaktierung von einem Biofluid mit einem Hydrogel mit der angegebenen Ladungs- und Netzwerkcharakteristik des Typs 1 gemäß Tabelle 5.1 können daher eine selektive Auswahl an Wachstumsfaktoren mit zu DKK1, bNGF, PDGF-BB, VEGF, sowie Chemokinen (bspw. Eotaxin, Gro-alpha, IL-8, IP-10, MCP-1 MIP-1 alpha, MIP1 beta, Rantes, SDF1 alpha) und Zytokinen mit zu IFN-Gamma, IL4, und Sclerostin strukturell ähnlichen Proteine entsprechend der angemerkten Sequenzmotive und Proteinfamilien aus dem Biofluid abgereichert oder durch Vorbeladung des Hydrogels in ihrer Konzentration im Biofluid erhöht werden, währenddessen die oben genannten Signalmoleküle EGF, FGF-2, TGFbl, PLGF, IL-10, IL1 beta, IL6, und TNF alpha vorteilhaft in ihrer Konzentration nahezu nicht verändert werden. Außerdem besteht bei diesem Hydrogel eine Wechselwirkung des GB1 mit den für die Blutgerinnung wichtigen Enzymen Thrombin und Antithrombin gemäß Uwe Freudenberg et al., "Journal of Controlled Release," Journal of Controlled Release : Official Journal of the Controlled Release Society 220, no. Part A (December 28, 2015): 79-88, doi:10.1016/j.jconrel.2015.10.028.

Ein weiteres besonders vorteilhaftes Ausführungsbeispiel (AB2) besteht in dem Hydrogeltyp UGB1-GB2 02 (siehe Tabelle 2) welches bis auf die geringere Bindung der zwei Chemokine MIP-1 alpha und MIP-1 beta und Sclerostin (jeweils ca. 40%, Tabelle 3) und der noch vorteilhafteren geringeren Bindung von IL-10 (15%), IL-6 (8%), TNF alpha (25%), siehe Tabelle 3 und der noch vorteilhafteren, nahezu keinen Bindung von FGF-2 (5%) und TGFbl (0%) und der fehlenden Wechselwirkung des GB2 mit den Blutgerinnungsenzymen Thrombin und Antithrombin (siehe Uwe Freudenberg et al., "Journal of Controlled Release," Journal of Controlled Release : Official Journal of the Controlled Release Society 220, no. Part A (December 28, 2015): 79-88, doi:10.1016/j.jconrel.2015.10.028) ein zum Hydrogeltyp UGB1:GB1 01 nahezu identisches Bindungs- bzw. Sequestrierungsmuster aufweist (siehe Tabelle 3). Ein weiteres vorteilhaftes Ausführungsbeispiel stellt UGB2-GB5 23 (AB 23, siehe Tabelle 1) dar, welches eine nach der in Tabelle 5.1 ebenfalls wie das AB2 dem Typ 2 zuzuordnende Ladungscharakteristik, jedoch mit einem leicht abweichenden Sulfonatgehalt von 0,14 mmol/ml (Tabelle 1 oder Tabelle 5.2 Spalte B) und einer leicht abweichender Anzahl von Sulfonatgruppen je Wiederholeinheit (WE) dividiert durch die Molare Masse (MW) der WE in [mol/g] von 0,0038 aufweist, wobei jedoch beide Parameter im in der Tabelle 5.1 vorgegebenen Rahmen für ein Hydrogel des TYPs 2 liegen. Das Hydrogel wurde jedoch aus einem anderen geladenen, synthetischen Gelbaustein (GB5) mit anderer Vernetzungsreaktion (Vernetzungstyp 2, Tabelle 1) gebildet und weist bis auf die etwas stärkere Bindung der zwei Chemokine MIP-1 alpha und MIP-1 beta ein nahezu identisches Bindungs- bzw. Sequestrierungsmuster wie das AB2 auf (siehe Tabelle 3). Dies bestätigt insofern die Einstufung der Hydrogele mit den charakteristischen Ladungsmustern nach Tabelle 5.1 und stellt ebenso ein besonders vorteilhaftes Sequestrierungsmuster dar.

Ein weiteres vorteilhaftes Ausführungsbeispiel (AB3) besteht in dem Hydrogeltyp UGB1-GB3 03 (siehe Tabelle 2), welches aufgrund der deutlich geringeren Konzentration an Sulfat- oder Sulfonatgruppen im Hydrogel (Tabelle 5.2, Spalte B, 0,06 im Vergleich zu 0,12 mmol/ml für AB1 oder AB2) und auch der niedrigeren Anzahl an Sulfat- bzw. Sulfonatgruppen je WE geteilt durch die Molmasse der WE von 0,0019 im Vergleich zu 0,005 oder 0,0035 für AB 1 oder AB 2 (siehe Tabelle 5.2, Spalte A) verglichen mit dem AB 1 und AB2 eine deutlich geringere Bindung zu vielen Proteinen (Signalmolekülen) aufweist. Bei diesem Hydrogeltyp wird bNGF, PDGF-BB, und VEGF A, Eotaxin, GRO-alpha, IP-10, Rantes, SDF-1 alpha, IFN-gamma und IL-4 zu mehr als 50% vom Hydrogel gebunden und daher aus einem Biofluid abgereichert, dagegen wird IL-8, MCP-1 und IL-12p40 nur mit ca. 45% (siehe Tabelle 3), HGF, MIP-1 alpha, MIP-1 beta, IL-1 beta, IL-10, IL-6, TNF alpha und DKK1 mit 16-36% nur gering (siehe Tabelle 3) und FGF-2, TGFbI, EGF, PLGF, GM-CSF und Sclerostin nahezu nicht (<12%) (siehe Tabelle 3) und daher die Mehrzahl der Signalmoleküle deutlich geringer als für das AB 1 und AB 2 vom Hydrogel gebunden. Mit diesem Geltyp ist beispielsweise eine weitgehend selektive Abtrennung von bNGF, PDGF-BB, und VEGF A, Eotaxin, GRO-alpha, IP-10, Rantes, SDF-1 alpha, IFN-gamma und IL-4 aus beliebigen Biofluids möglich.

Ein weiteres vorteilhaftes Ausführungsbeispiel besteht im Hydrogel UGB1-GB4 04 (siehe Tabelle 1), welches aufgrund der hohen Konzentration an Sulfat- oder Sulfonatgruppen von 0,28 mmol/ml im Hydrogel (Tabelle 5.2, Spalte B) und einer ebenfalls hohen Anzahl an Sulfonatgruppen je WE geteilt durch die Molmasse der WE von 0,0045 mol/g bis auf HGF, PLGF, GM-GSF und EGF alle untersuchten Faktoren mit einer hohen Effektivität (zwischen 72-100%, siehe Tabelle 3) im Hydrogel bindet und somit aus dem Biofluid abreichert (siehe Tabelle 3).

Insbesondere findet mit diesem Hydrogel eine Abreicherung aller untersuchten und strukturell ähnlicher Faktoren (IEP und das Vorhandensein von Strukturähnlichkeiten), insbesondere der Klasse der Chemokine, welche eine hohe strukturelle Ähnlichkeit der Tertiärstruktur aufweisen, welche durch die Interaktion von vier Cysteinen durch Disulfidbrücken (entsprechend PROSITE ID: PS00471 und PS00472), durch eine stark positive Nettoladung IEP >9 oder positiv geladene Domänen wie bei MIP1 alpha und MIP1 beta und einem Molekulargewicht von unter 10 kDa (siehe Tabelle Proteineigenschaften) charakterisiert sind, FGF-2 (IEP 9,58), TGFbI (IEP 8,59) sowie strukturell ähnlichen Proteinen der FGF Familie (entsprechend PROSITE ID: PS00247), der TGF Familie (entsprechend PROSITE ID: PS00250) sowie der Klasse der Zytokine zuzuordnenden Signalmoleküle mit leicht basischem IEP wie IL-10 (IEP 7,65) sowie Signalmoleküle mit einem IEP im Neutralbereich (pH 5,5-7) wie IL1$\beta$, IL6, und TNF und strukturell ähnlichen Proteinen der IL-1$\beta$ Protein Familie (entsprechend PROSITE ID: PS00253) und der IL-10 Protein Familie (entsprechend PROSITE ID: PS00520), aus angrenzenden Biofluids statt. Ein weiteres vorteilhaftes Ausführungsbeispiel, UGB2-GB4 20 (siehe Tabelle 1), welches mit einer nach der in Tabelle 5.1 ebenfalls wie das AB4 dem Typ 4 zuzuordnenden Ladungscharakteristik, welches den gleichen GB 5, siehe Tabelle 5.1 Spalte A: 0,0045, aber eine geringere noch im Rahmen des Typ 4 zuzuordnende Sulfonatkonzentration von 0,16 mmol/ml aufweist(siehe Tabelle 1 und Tabelle 5.1), dabei jedoch mit abweichender Vernetzungsreaktion (VN: 2 in Tabelle 1) gebildet wurde, weist ein nahezu identisches Bindungs- bzw. Sequestrierungsmuster wie das UGB1-GB4 04 auf (siehe Tabelle 3). Dieser Befund bestätigt somit ebenfalls die wirksame Vorhersagekraft der Ladungscharakteristik der Typen 1-5 gemäß Tabelle 5.1 für die differenzierte Sequestrierung von Stoffen oder Stoffgruppen.

Ein weiteres Ausführungsmuster ist das aus UGB1 und UGB3 gebildete ungeladene Hydrogel (UGB2 - UGB3 26), welches erwartungsgemäß als Negativkontrolle nahezu keines der Signalmoleküle aus dem Biofluid bindet. Bis auf die aufgrund der großen Standardabweichungen als nicht signifikant einzustufende geringe Sequestrierung von PDGF-BB in Höhe von 19,7 ± 23,1% und IP 10 in Höhe von 16,1 ± 16,4 % findet keine Sequestrierung (≤ 1,3% für alle getesteten Signalmoleküle, siehe Tabelle 3) durch dieses Hydrogel statt. Dieser Befund kann ganz klar auf die Abwesenheit von geladenen Affinitätszentren und somit auf die Abwesenheit der hier diskutierten Ladungswechselwirkungen zurückgeführt werden. Das Speichermodul und somit auch die Netzwerkmaschenweite ist dagegen mit 4,4 kPa (Tabelle 1) nahezu identisch mit dem Speichermoduli der UGB1-GB2 02, UGB2-GB4 20 und UGB2-GB5 23, d.h. es liegen zu den geladenen Ausführungsbeispielen vergleichbare sterische Wechselwirkungen mit den Signalmolekülen vor. Durch diese Ergebnisse, insbesondere auch durch die fehlende Wechselwirkungen (d.h. die Abwesenheit von Bindungs- und Sequestrierungseffekten) des ungeladenen Hydrogeles (UGB2-UGB3 26) mit den Signalmolekülen werden die Ansprüche entsprechend Tabelle 5.1 zur differenzierten Sequestrierung untermauert.

Gleichzeitig können aber einzelne Faktoren selektiv durch gezielte Vorbeladung der Hydrogele der Typen 1 bis 3 der Tabelle 5 parallel und unabhängig zur Sequestrierung und somit Abreicherung der anderen Faktoren in das Biofluid freigesetzt werden. Somit können mit den verschiedenen Hydrogeltypen nahezu beliebige Spiegel einzelner Signalmoleküle durch gezielte Vorbeladung der Hydrogele oder aber die nahezu quantitative Sequestrierung (Abreicherung) von Signalmolekülen (bspw. Anwendung zur Abtrennung von Signalmolekülen aus beliebigen proteinhaltigen Lösungen) in komplexen Biofluids eingestellt werden.

Aufgrund der Ausführungsbeispiele in UGB1-GB1 01 bis UGB2-UGB3 26 sind die folgenden für die Beschreibung der Erfindung wichtige Zusammenhänge zwischen Proteineigenschaften, Hydrogeleigenschaften und Bindung der Proteine im Hydrogelnetzwerk ersichtlich: Eine starke Bindung der Signalmoleküle entsprechend der Tabelle 3 korreliert auf der Seite der Proteine mit einer positiven Nettoladung (und hier mit einem hohem, basischen IEP als entsprechenden Parameter) sowie invers mit dem Molekulargewicht (hoher IEP und kleines Molekulargewicht verstärken die Bindung, siehe Tabelle 3, die stark positiv geladenen und gleichzeitig relativ kleinen Chemokine (<9 kDa) werden am stärksten gebunden und auf der Seite der Hydrogelnetzwerke wirkt eine (1) hohe Konzentration an Sulfat- oder Sulfonatgruppen im Hydrogel (Tabelle 1 und 5.2, Spalte B) und (2) eine hohe Anzahl an Sulfat- bzw. Sulfonatgruppen je WE geteilt durch die Molmasse der WE verstärkend auf die Bindung der Signalmoleküle. Entsprechend dieser Parameter ergibt sich für die Hydrogele folgende Reihung von der stärksten Bindung UGB1-GB4 04 ≈ UGB2-GB4 20 <UGB1-GB1 01<UGB1-GB2 02 ≈ UGB2-GB5 23 <UGB1-GB3 03<<UGB2-UGB3 26 zur schwächsten Bindung, dieser Befund wird durch die experimentell gefundene Bindungswerte untermauert (siehe Tabelle 3). Darüber hinaus sind durch unterschiedliche Kombinationen beider zuvor genannter Hydrogelnetzwerkparameter (1) und (2) auch die in der Literatur als "spezifische Wechselwirkungen" - d.h. ein räumliches Matching der unterschiedlichen Wechselwirkungszentren, bekannten starken Wechselwirkungen zwischen Proteinen und negativ geladenen Polyelektrolyten (bspw. Glykosaminoglykanen) einstellbar und somit können auch auf der Proteinseite andere strukturelle Eigenschaften jenseits von Molekulargewicht und Nettoladung genutzt werden, um die Bindung zu den Hydrogelen zu modulieren. Diese Zusammenhänge erklären auch die geringe Bindung der stark basischen und relativ kleinen Signalmoleküle FGF-2, TGFb1 und PLGF zu den Hydrogeltypen UGB1-GB1 01, UGB1-GB2 02, UGB1-GB3 03 und UGB1-GB4 04.

Weitere Kontrollmöglichkeiten der Spiegel löslicher Signalmoleküle bestehen in der sequentiellen oder kombinierten Anwendung der unterschiedlichen Hydrogeltypen, um eine breite Vielfalt von spezifischen Sequestrierungs- und Freisetzungseigenschaften präzise einstellen zu können. Durch gezielte Variation der Netzwerkeigenschaften (insbesondere die zuvor beschriebenen Parameter (1) und (2) und auch der Kombination unterschiedlich zusammengesetzter Hydrogelmikropartikel (bspw. Beladung mit einzelnen Signalmolekülen) in sogenannten mehrphasigen (d.h. durch Mischung von Hydrogeltypen unterschiedlichen Typs oder Vorbeladung mit Signalmolekülen oder Proteinen) Hydrogelmaterialien können auch andere vorteilhafte Konzentrationsverhältnisse in angrenzenden Biofluids eingestellt werden.

Durch eine Erweiterung der Analyse für weitere Signalmoleküle ist mit dem vorgeschlagenen Verfahren auch die Kontrolle der Konzentration dieser Stoffe in beliebigen Biofluids möglich.

Um die komplexen biologischen Funktionen von Signalmolekülen, beispielsweise für therapeutische Konzepte, effektiv kontrollieren zu können, ist neben der nachhaltigen Freisetzung einzelner Signalmoleküle aus mit den Signalmolekülen vorbeladenen Hydrogelen ein aktives Management verschiedener Signalmoleküle aus der unmittelbaren biologischen Umgebung der Zellen bzw. der biologischen Anwendung notwendig. Es sind also ganzheitlich die Wechselwirkungen mit einer Vielzahl relevanter Signalmoleküle zu betrachten. Wenn Hydrogele mit sulfatieren oder sulfonierten Komponenten beispielsweise mit einem Signalmolekül zur nachhaltigen Freisetzung beladen sind, können gleichzeitig mit der therapeutisch gewünschten Freisetzung der Signalmoleküle andere ebenfalls therapeutisch wichtige Moleküle aus der biologischen Umgebung sequestriert und somit inaktiviert werden, also einen unerwünschten Nebeneffekt darstellen. Andererseits können aber auch gezielt therapeutisch unerwünschte Signalmoleküle in das sulfatierte bzw. sulfonierte Gruppen tragende Hydrogel sequestriert und somit inaktiviert werden. Insgesamt sind sehr komplexe Freisetzungs- und Sequestrierungszenarien möglich, die in ihrer Gesamtheit über die biologische Wirkung, insbesondere die molekularen Trenneigenschaften, der Materialien entscheidet.

Weitere vorteilhafte Ausgestaltungen der Erfindung bestehen in der Anwendung von Hydrogelen entsprechend der Typen 1-4 der Tabelle 5 zur Modulation der Konzentration beziehungsweise Spiegel von biologisch aktiven Proteinen in niedrigen Gesamtspiegeln.

Ein wesentlicher Vorzug des Verfahrens besteht in der Universalität seiner Einsatzmöglichkeiten. Das Verfahren ist sowohl zur biotechnologische Aufreinigung sowie zur Trennung von Proteingemischen aus oder in Biofluiden mittels Einsatz von Hydrogelen mit sulfatierten bzw. sulfonierten Komponenten vorteilhaft anwendbar.

In beispielhaftem engeren Sinne ist das Verfahren zum Faktormanagement *in vivo* zur Kontrolle der Angiogenese, von Immunerkrankungen, von Diabetis, neurodegenerativen Erkrankungen und der Wundheilung einsetzbar.

Bei der Wundheilung werden die zumeist pro-inflammatorisch wirkenden Chemokine (bspw. Eotaxin, GRO-a, IL-8, IP-10, MCP-1, MCP-3, MCD, Rantes und SDF-1) im Inneren der Hydrogele sequestriert, währenddessen pro-regenerative Faktoren (bspw. EGF, FGF-2, TGFbl, IL-10, HGF und PLGF) weitgehend unbeeinflusst bleiben.

Ein besonders wichtiges Anwendungsgebiet der Erfindung besteht in der Kontrolle der Konzentrationen von Stoffen in Biofluiden, welche für Zellschicksalsentscheidungen in vitro und in vivo verantwortlich sind. Die Fehlregulation pro-inflammatorisch wirkender Chemokine und die damit einhergehende chronische Entzündung ist ursächlich für die Entwicklung verschiedener Erkrankungen wie Morbus Crohn, Colitis ulcerosa, Multiple Sklerose, Asthma oder Rheumatoide Arthritis. Die gezielte Modulation der Konzentration dieser Entzündungsfaktoren aus Biofluids durch die erfindungsgemäße Anwendung unterschiedlicher Hydrogele mit sulfatierten bzw. sulfonierten Komponenten stellt dabei ein mögliches Anwendungsszenario dar.

Im weiteren Sinne erlaubt die erfindungsgemäße Anwendung der Hydrogele mit sulfatierten bzw. sulfonierten Komponenten eine gezielte Aufreinigung von den hier untersuchten oder strukturell ähnlichen Signalmolekülen aus komplexen Proteingemischen. Die beschriebenen Gelsysteme können somit zur gezielten Aufreinigung von Proteinen aus Zelllysaten mikrobieller oder eukaryotische Herkunft biotechnologisch eingesetzt werden. Dabei werden besagte Zelllysate durch erfindungsgemäße Hydrogele geleitet, um die Signalmoleküle zu binden. In einen zweiten Schritt können die gebundenen Stoffe von den Hydrogelen zur weiteren Verwendung durch Spülung mit Hochsalzlösungen oder positiv geladenen Polyelektrolyten (z.B. Chitosan) wieder entfernt und somit abgetrennt werden. Des Weiteren ist eine Negativselektion zur Abtrennung von den unter besagten Bedingungen gering an die Hydrogele der Typen 1-3 bindenden bspw. EGF, FGF-2, TGF-$\beta$, IL-10, HGF und PLGF Signalmolekülen durch Nutzung von Überständen nach 24h Bindung möglich.

Ein relevanter praktischer Vorteil der Erfindung besteht darin, dass auch bei hohen Albuminkonzentrationen von 1 mg/ml bis 45 mg/ml bei gleichzeitig niedrigen Spiegeln an Signalmolekülen von 100 pg/ml bis 2000 ng/ml in physiologischem Elektrolyt als Biofluid, die Bindung einer Vielzahl von biologisch relevanten Signalmolekülen zu sulfatierten oder sulfonierten Hydrogelen mit gezielt abgestufter Ladungscharakteristik und Netzwerkstruktur realisiert wird und darauf aufbauend Selektivitätsunterschiede in der Bindung der einzelnen Signalmoleküle zu den unterschiedlichen Hydrogeltypen genutzt werden können.

Die in der Tabelle 1 angeführten Ausführungsbeispiele von UGB1-GB1 01 bis UGB1-GB4 011 sind kovalent vernetzte Hydrogele gemäß Vernetzungsreaktion 1, bei denen 6 bis 24 Carboxylgruppen (je nach Molverhältnis 1,5 bis 6 bei vierarmigem SternPEG) eines Moleküls des GB4 (Poly(4-Styrensulfonsäure-co-Maleinsäure, Molverhältnis Styrensulfonsäure: Maleinsäure 3:1, Tabelle 1) mittels EDC/sulfo-NHS aktiviert wurden und direkt mittels dem vierarmigen, aminterminierten UGB1 zu einem Hydrogel umgesetzt wurden (siehe Tabelle 1). Dadurch resultieren Hydrogele mit variabler Sulfonatkonzentration von 0,28 bis 0,06 mmol/ml sowie variable Speichermoduli von 4,8 bis 19,6 kPA (siehe Tabelle 1).

Die in der Tabelle 1 angeführten Ausführungsbeispiele UGB1-GB5 12 bis UGB1-GB5 19 sind kovalent vernetzte Hydrogele nach Vernetzungsprinzip 1, bei denen 6 bis 24 Carboxylgruppen (je nach Molverhältnis 1,5 bis 6 bei vierfunktionalen SternPEG) eines Moleküls der Poly(4-Styrensulfonssäure-co-Maleinsäure) mit dem Molverhältnis Styrensulfonsäure zu Maleinsäure 1:1 (GB5), mittels EDC/SNHS aktiviert wurden und direkt mittels dem vierarmigen, aminterminierten UGB1 zu einem Hydrogel umgesetzt wurden (siehe Tabelle 1). Dadurch resultieren Hydrogele mit variabler Sulfonatkonzentration von 0,13 bis 0,05 mmol/ml sowie variablen Speichermoduli von 3 bis 21,8 kPA (siehe Tabelle 1).

Die Ausführungsbeispiele UGB2-GB4 20 bis UGB2-GB4 22 sind kovalent vernetzte Hydrogele nach Vernetzungsprinzip 2, bei denen acht der mittels EDC/sulfo-NHS aktivierten Carboxylgruppen des GB4 (Poly(4-Styrensulfonsäure-co-Maleinsäure) mit dem Molverhältnis Styrensulfonsäure : Maleinsäure 3:1) in einem ersten Schritt mittels des kurzen bifunktionellen Vernetzermoleküls N-(2-Aminoethyl)maleimid funktionalisiert und anschließend aufgereinigt und isoliert wurden. Diese Derivate des GB4 wurden anschließend durch Vermischen in physiologischer Kochsalzlösung (oder Serum) mit dem UGB-2 (thiolterminiertem vierarmigen PEG) zu einem Hydrogel umgesetzt. Es ergeben sich Hydrogele mit einer Abstufung in der Konzentration der Sulfonatgruppen von 0,16 bis 0,05 mmol/ml und der Speichermoduli von 3,7 - 0,2 kPa.

Die Ausführungsbeispiele UGB2-GB5 23 bis UGB2-GB5 25 sind kovalent vernetzte Hydrogele nach Vernetzungsprinzip 2 (VN2, Tabelle 1), bei denen acht der mittels EDC/sulfo-NHS aktivierten Carboxylgruppen des GB5 (Poly(4-Styrensulfonssäure-co-Maleinsäure) mit dem Molverhältnis Styrensulfonssäure : Maleinsäure 1:1) in einem ersten Schritt mittels des kurzen bifunktionellen Vernetzermoleküls N-(2-Aminoethyl)maleimid funktionalisiert und anschließend aufgereinigt und isoliert wurden. Diese Derivate des GB5 wurden anschließend durch Vermischen in physiologischer Kochsalzlösung (oder Serum) mit dem UGB-2 (thiol-terminierten vierarmigen PEG) zu einem Hydrogel umgesetzt. Es ergeben sich Hydrogele mit einer Abstufung in der Konzentration der Sulfonatgruppen von 0,14 bis 0,04 mmol/ml und der Speichermoduli von 4,6 - 0,3 kPa.

Das Ausführungsbeispiel UGB2-UGB3 26 ist ein ungeladenes PEG-Hydrogel, bei dem nach Vernetzungsprinzip 2 thiolterminiertes vierarmiges PEG (UGB-2) mit einem Maleimid-terminierten vierarmigen PEG (UGB-3) umgesetzt wurde.

Dieses Hydrogel diente in den Sequestrierungsversuchen als ungeladene Negativkontrolle.

Anhand der Ausführungsbeispiele 04 - 5 sind überaschenderweise vollsynthetische Hydrogele mit einer ganzen Bandbreite vorteilhafter Eigenschaften und Eigenschaftskombinationen ohne nachteilige immunogenen Reaktionen darstellbar. So ist eine breite Konzentration von Sulfonatgruppen im gequollenen Hydrogel von 0,04 bis 0,28 mmol/ml und mit einer ebenso großen Variation des Speichermodulus 0,2 bis 21,8 kPa weitgehend unabhängig voneinander einstellbar. Zum Beispiel weisen die Hydrogele UGB2-GB4 21, UGB1-GB5 14, UGB1-GB5 15 und UGB1-GB4 08 eine gleichbleibende Konzentration an Sulfonatgruppen im gequollenen Hydrogel von 0,08 mmol/ml aber gleichzeitig steigende Speichermoduli von 1,1, 5,5, 12,5 und 19,6 kPa auf, d.h. die Sulfonatkonzentration und die Steifigkeit der Hydrogele können über einen weiten Bereich unabhängig voneinander moduliert werden. Ebenfalls können mit der Auswahl des UGB1-GB5 19 mit einer Sulfonatkonzentration von 0,05, des UGB1-GB4 11 mit einer Sulfonatkonzentration von 0,06 und des UGB1-GB4 10 mit einer Sulfonatkonzentration von 0,09 bei einem nahezu konstanten Speichermodulus von 8,0-8,5 kPa die Sulfonatkonzentration unabhängig von der Steifigkeit der Hydrogele variiert werden (siehe Tabelle 1). Außerdem sind durch eine weitere Variation der Molverhältnisse UGB:GB in den Bereichen zwischen 1,5 und 3 sowie zwischen 3 und 6 für die VN1 und zwischen 0,5 und 0,75 sowie 1 und 1,5 sowie 1,5 und 2 für die VN2 und eine weitere Variation der Konzentration der GB und UGB noch eine Vielzahl anderer Eigenschaftskombinationen darstellbar.

Überraschenderweise konnten dabei durch die Vernetzung der Poly(4-Styrensulfonssäure-co-Maleinsäure), startend mit der EDC/sulfoNHS Aktivierung einer der eng benachbarten Säuregruppen in der Maleinsäure, welche in ihrer Reaktivität nicht nur durch die benachbarte Carboxylgruppe sondern insbesondere durch die Sulfonatgruppen und die hydrophoben Styreneinheiten in einer nicht vorhersagbaren Weise beeinflusst werden, mit ungeladenen Bausteinen definierte Hydrogelmaterialien mit den zuvor genannten weit abstufbaren Eigenschaften synthetisiert werden. Hier wurde eine präzise Netzwerkbildung (für VN1) oder Derivatisierung (für VN2) durch die Anwendung einer überaschenderweise sehr geringen Aktivierungszeit von 1 min, das heißt der Zeit, in der das EDC/sulfoNHS zur Poly(4-Styrensulfonssäure-co-Maleinsäure) gegeben wurde, siehe Tabelle 4, erreicht, bevor im Anschluss ein Amingruppen tragendes Molekül durch Mischung mit der dann aktivierten Poly(4-Styrensulfonssäure-co-Maleinsäure) und einer sehr intensiven Durchmischung der Reaktionspartner umgesetzt wurde.

In einem weiteren Ausführungsbeispiel wurden Hydrogele der Typen UGB1-GB1 01, UGB1-GB4 20, UGB1-GB5 23 und UGB1-GB3 26 mit dem Adhäsionspeptid CWRGDSP funktionalisiert und mit VEGF-A und FGF-2 vorbeladen und für die Kultivierung humaner Endothelzellen auf der Geloberfläche in einem serumfreien Zellkulturmedium genutzt. Die Morphologie der adhärierten Zellen wurde nach 24h Kulturzeitraum analysiert, wie in der Fig. 1 für UGB1-GB1 01, UGB2-GB4 20, UGB2-GB5 23, UGB2-UGB3 26 gezeigt. Die Endothelzellen nahmen dabei auf den unterschiedlichen Hydrogelen eine unterschiedliche Morphologie, beschreibbar durch die beiden Parameter Aspektverhältnis und die Zirkularität, ein. Ein großes Aspektverhältnis und eine geringe Zirkularität entspricht der gewünschten elongierten Endothellzellmorphologie, dem ersten Schritt zur Bildung der biologisch gewünschten tubulären Strukturen. Das Aspektverhältnis der Endothelzellen, kultiviert auf UGB1-GB1 01, UGB2-GB4 20 und UGB2-GB5 23, betrug 3,4 ± 1,0, 4,1 ± 1,1 und 4,9 ± 1,6, währenddessen die ungeladenen Referenzgele UGB2-UGB3 26 ein deutlich geringeres Aspektverhältnis von 2,1 ± 1,2 aufwiesen. Die Zirkularität verhielt sich entsprechend invers mit 0,5 ± 0,1, 0,4 ± 0,1, 0,3± 0,1 und 0,7± 0,3 für Endothelzellen auf den Hydrogelen UGB1-GB1 01, UGB1-GB4 20 und UGB1-GB5 23 und UGB1-GB3 26. Diese Unterschiede waren statistisch hochsignifikant. Dementsprechend verhalten sich die Endothelzellen auf den Hydrogelen mit den entsprechend Tabelle 5.1 unterschiedlichen Ladungs- und Sequestrierungseigenschaften des Typs 1 (UGB1-GB1 01), des Typs 4 (UGB2-GB4 20), des Typs 2 (UGB2-GB5 23) und des ungeladenen Typs 5 (UGB1-GB3 26) deutlich unterschiedlich. Auf dem ungeladenen Hydrogel zeigen die Zellen unerwünschte kurze Aspektverhältnisse und eine hohe Zirkularität, während die gewünschte Morphologie in der Reihenfolge UGB1-GB1 01< UGB1-GB4 20 < UGB2-GB5 23 zunimmt. Das Hydrogel UGB2-GB5 23 des Typs 2 zeigt in diesem Versuch die besten Ergebnisse. Diese Abstufung bestätigt insofern einen direkten Einfluss der Ladungseigenschaften und Sequestrierungsmuster auf die Kultur humaner Endothelzellen.

In einem weiteren Ausführungsbeispiel wurden Hydrogele des Typs 1 (UGB1-GB1 01), des Typs 4 (UGB1-GB4 20), des Typs 2 (UGB1-GB5 23) und des ungeladenen Typs 5 (UGB1-UGB3 26) zur Einpolymerisation humaner mesenchymaler Stromazellen genutzt. Die Hydrogele waren zellresponsiv, das heißt, neben der Funktionalisierung der Hydrogele mit dem die Adhäsion vermittelnden Peptid CWGRGDSP wurde die enzymatisch durch von diesen Zellen sezernierte Matrixmetalloproteasen spaltbare Peptidsequenz GCGGPQGIWGQGGCG an den jeweiligen ungeladenen Baustein vorkonjugiert und zur Vernetzung gemäß VN2 genutzt. Nach 24 Stunden wurde die metabolische Aktivität der eingebetteten Zellen als Viabilitätsnachweis mittels eines PrestoBlue®-Tests charakterisiert. Dabei betrug die metabolische Aktivität, gemessen als relative Fluoreszenzeinheiten, der einpolymerisierten humanen mesenchymalen Stromazellen innerhalb des Hydrogels des Typs 1 7112 ± 3924, des Typs 2 3704 ± 2945, des Typs 4 3160 ± 6023 und des ungeladenen Typs 5 2316 ± 446. Entsprechend zeigten humane mesenchymalen Stromazellen, eingebettet in Gele des Typs 2, die höchste und, eingebettet in Gele des ungeladenen Referenzgels, die geringste metabolische Aktivität. Die Hydrogele, insbesondere des Typs 2, erwiesen sich entsprechend als besonders vorteilhaft für die Kultivierung lebender humanen mesenchymalen Stromazellen in 3D.

In einem weiteren vorteilhaften Ausführungsbeispiel wurden UGB1-GB1 01, UGB2-GB4 20, UGB2-GB5 23, UGB2-UGB3 26 mit dem positiv geladene Gruppen tragendem Antibiotikum Gentamicin durch differenzierte Sequestrierung funktionalisiert. Im Anschluss wurde die Inhibierung durch Freisetzung des Gentamicin aus den unterschiedlichen Hydrogelen anhand der zwei relevanten, krankheiteregenden Bakterienstämme Escherichia coli (*E. coli*) und Staphylococcus epidermidis (*Staph. epidermidis*) gemessen. Die antimikrobielle Wirkung wurden anhand der Größe der Inhibitionszone (Entfernung vom Hydrogel auf der Kulturplatte, siehe Material und Methoden) gemessen. Es ergaben sich für die Hydrogele der Typen UGB1-GB1 01, UGB2-GB4 20, UGB2-GB5 23, UGB2-UGB3 26 ohne vorherige Gentamicinsequestrierung keine nachweisbaren Inhibitionszonen, d.h. die Hydrogele wirkten nicht antimikrobiell. Im Falle der vorherigen differenzierten Sequestrierung von Gentamicin durch die Hydrogele wurden folgende abgestuften Inhibierungszonen vermessen: UGB1-GB1 01: 3,08 ± 0,33 mm, UGB2-GB4 20 *E. coli*: 3,08 ± 0,33 mm, *S. epidermidis:* 2,94 ± 0,35 mm, UGB2-GB4 20: *E. coli:* 1,78 ± 0,27 mm, *S. epidermidis:* 1,76 ± 0,45 mm; UGB2-GB5 23 *E. coli:* 2,18 ± 0,38 mm, *S. epidermidis:* 1,81 ± 0,51 mm und für die ungeladene Referenzgele UGB2-UGB3 26 *E. coli:* 0,48 ± 0,36 mm, *S. epidermidis:* 0,90 ± 0,28 mm. Dementsprechend werden für die ungeladenen Referenzgele UGB2-UGB3 26 erwartungsgemäß nur geringste Mengen an Gentamicin unspezifisch sequestriert und anschließend wieder freigesetzt. Für die anderen Hydrogele konnte eine abgestufte antimikrobielle Wirkung in der Reihenfolge UGB2-GB4 20 < UGB2-GB5 23 < UGB1-GB1 01 gefunden werden. Dieser Befund unterstützt die Ansprüche zur differenzierten Sequestrierung von positiv geladenen Wirkstoffmolekülen. Außerdem können durch Variation der Sequestrierungskonzentration an Gentamicin auch unterschiedliche antimikrobielle Wirkungen eingestellt werden.

**Material und Methoden:**

**Regio-selektive Desulfatierung des Heparins (Herstellung von GB2 und GB3 aus GB1)**

Zur Synthese regio-selektiv desulfatierten Heparins (GB2 und GB3) wurde zuerst Heparin (MW 14.000, Merck-Millipore , Hersteller-Nr.: 375095, Germany, GB1) in deionisierten, ultrareinem Wasser gelöst und mit Hilfe einer Amberlite IR-120 H+ Ionenaustauschsäule (Sigma-Aldrich, Deutschland) entsalzen. Durch Zugabe von Pyridin (Sigma-Aldrich, Deutschland) zur Heparin-Lösung bis zum Erreichen von pH 6 wurde ein Heparin-Pyridin-Salz geformt, welches durch Lösungsmittelverdampfung in einem B-490 Rotationsverdampfer (Büchi, Deutschland) angereichert, anschließend lyophilisiert bei -80°C (GEA Lyovac GT2, Deutschland) und bis zur weiteren Verarbeitung bei -20°C gelagert wurde. Zur Herstellung von N-desulfatierten Heparin **(N-DSH, GB2)** wurde 1 g/l Heparin-Pyridin in einem Gemisch aus DMSO und deionisierten, ultrareinem Wasser (95:5) gelöst und bei 50°C für 1,5 Stunden inkubiert. Die entstandene Lösung wurde 1:1 mit deionisierten, ultrareinem Wasser verdünnt und mit 1M Natriumhydroxid-Lösung (Sigma-Aldrich, Deutschland) auf pH 9 eingestellt. Nach der Dialyze der Lösung (Spectrum Labs, Deutschland, MWCO= 8 kDa) gegen deionisiertes, ultrareines Wasser für drei Tage, erfolge die N-Acetylierung des desulfatierten Heparins. Dazu wurde zur Lösung 10% v/v Methanol (Sigma-Aldrich, Deutschland) und 50 mM Natriumcarbonat (Sigma-Aldrich, Deutschland) hinzugefügt. Die Acetylierungsreaktion erfolgte für drei Stunden unter Kühlung auf 4°C und ständigen Rühren bei 400 rpm durch halbstündliche Zugabe von 400 $\mu$l Acetanhydrid (Sigma-Aldrich, Deutschland) pro 1 mg Heparin und anschließender Einstellung des pH auf 7.5 durch Zugabe einer 2 M NatriumcarbonatLösung. Zur Herstellung von 6O-N-desulfatierten Heparin **(6ON-DSH, GB 3)** wurde 1 g/l Heparin-Pyridin in einem Gemisch aus DMSO und deionisierten, ultrareinem Wasser (95:5) gelöst und bei 90°C für 24 Stunden inkubiert. Die entstandene Lösung wurde 1:1 mit deionisierten, ultrareinem Wasser verdünnt und mit 1 M Natriumhydroxid-Lösung (Sigma-Aldrich, Deutschland) auf pH 9 eingestellt. Nach der Dialyze in einem in einem Dialyseschlauch (Spectrum Labs, Deutschland, MWCO= 8 kDa) gegen deionisiertes, ultrareines Wasser für drei Tage, wurde das 6O-N-desulfatierten Heparin durch Lösungsmittelverdampfung B-490 (Büchi, Deutschland) angereichert und anschließend lyophilisiert (GEA Lyovac GT2, Deutschland).

**Charakterisierung der GB und UGB (siehe Tabelle 2):**

Das Molekulargewicht der GB 1-GB3 wurde bestimmt mittels Mehrwinkellichtstreuung bei 690 nm einem Dawn HELEOS II (Wyatt Technology Europe, Deutschland). Für die Molgewichtsbestimmung benötigten dn/dc Werte wurden mit einem RI Detektor (Optilab T-rEX, Wyatt, Germany) bei einer Wellenlänge von $\lambda$ = 690 nm bestimmt. Die RI-Inkremente, dn/dc von 0.1351 mL g-1 für GB1, GB2, u. GB3 wurden für die Auswertung der Lichtstreuexperimente benötigt. Die Auswertung der Lichtstreuergebnisse zur Bestimmung der Molmasse erfolgte mit der Astra Software, Version 6.1 (Wyatt Technology, USA). Der Sulfatierungsgrad der Heparin-Derivate (GB1-GB3) wurde mittels Elementaranalyse (Elementar, Vario MICRO cube, Deutschland) auf Basis des molaren Verhältnisses S:N Verhältnisses bestimmt. Auf Grund das jede Disaccharideinheit genau ein N Atom enthält wurde über das molare Verhältnis von S:N der Sulfatierungsgrad von GB1, GB2 und GB3 (Anzahl von Sulfatgruppen/Wiederholeinheit) bestimmt. Weiterhin wurde daraus das Molekulargewicht der Wiederholeinheit bestimmt. Darauf basierend wurde die Anzahl an Sulfatgruppen pro Wiederholungseinheit bzw. pro Mol Polymer berechnet.

Das Molekulargewicht sowie die Anzahl an Sulfonatgruppen pro Wiederholungseinheit bzw. pro Mol Polymer des GB4 und GB 5 (Poly(4-Styrensulfonsäure-co-Maleinsäure)) basiert auf Herstellerangaben (Sigma-Aldrich, Hersteller-Nr.: 434566, Deutschland).

Das Molekulargewicht und die Struktur des UGB1, UGB2, UGB3, amino-, thiol- oder maleimid terminiertes vierarmiges PEG basiert auf Herstellerangaben (Jenkem technology, USA). Das Molekulargewicht und die Struktur des UGB4 (mit enzymatisch spaltbarem Peptid terminiertes vierarmiges PEG) ist nach der Prozedur, wie bei Tsurkan et al. 2013 (Adv. Mater. 2013, 25, 2606-2610) beschrieben, hergestellt und eingesetzt worden.

**Herstellung der Hydrogele basierend auf Heparin oder Heparinderivaten** (siehe auch Tabelle1): Zur Herstellung der Hydrogele wurden entsprechend des Hydrogeltyps (siehe Tabelle 2) als geladener Baustein (GB) Heparin (GB1) (Merck-Millipore, Hersteller-Nr.: 375095, Germany), desulfatierte Heparin-Derivate auf Basis von GB1 durch zuvor beschriebene Verfahren, siehe regioselektive Desulfatierung, für GB2 und GB3) in deionisierten, ultrareinem Wasser bei 4°C für 30 Sekunden bei 500 U/Min mit einem Vortexmischer (IKA, Germany) gelöst. Zusätzlich wurden für alle Hydrogeltypen als UGB1 vieramiges, aminterminiert PEG (MW 10.000, Jenkem technology, USA), 1-Ethyl-3-(3-dimethylaminopropyl)carbodiimid (EDC; Sigma-Aldrich, Deutschland) und N-hydroxysulfosuccinimide (sulpho-NHS; Sigma-Aldrich, Deutschland) in gleicher Weise gelöst. Um die vollständige Lösung der Hydrogel-Bausteine zu gewährleisten, wurden diese Lösungen zusätzlich für 5 min bei 4°C mit einem Ultraschallbad (RK 100H, Bandelin, Germany) behandelt. Zur Aktivierung des GB 1 bis GB 3 wurden EDC und sulpho-NHS (2:1 Verhältnis von EDC:sulpho-NHS) zum gelösten GB (4 mol sulpho-NHS, 8 mol EDC bezogen je mol UGB1 der fertigen Reaktionsmischung) hinzugefügt und für entsprechend der Zeiten in Tabelle 4 bei 4°C inkubiert. Nach der Aktivierungszeit des GB1, GB2, GB3 wurde zusätzlich das gelöste amino-terminierte 4-ArmPEG (UGB1) hinzugefügt und für 30 Sekunden bei 500 U/Min mit einem Vortexmischer (IKA, Germany) gemischt.

**Herstellung der Hydrogele basierend auf Poly(4-Styrensulfonsäure-co-maleinsäure) nach Vernetzungsreaktion 1 (VN1, siehe auch Tabelle1):**

Poly(4-Styrensulfonsäure-co-maleinsäure) mit dem Molverhältnis 4-Styrensulfonsäure:Maleinsäure 3:1 (Sigma-Aldrich, Hersteller-Nr.: 434566, Deutschland, GB4, siehe Tabelle 2) und mit dem Molverhältnis 4-Styrensulfonsäure:Maleinsäure 1:1, Sigma-Aldrich, Hersteller-Nr.: 434558, Deutschland, GB5, siehe Tabelle 2) und dem UGB1 (vierarmiges PEG, aminterminiert, (MW 10.000, Jenkem technology, USA) wurden genutzt, um kovalent vernetzte Hydrogele wie folgt herzustellen: GB4 oder GB5 und UGB1 wurden jeweils in einem Drittel des Gesamtvolumens der Hydrogelmischung in deionisierten, ultrareinem Wasser mit der 3fachen Konzentration wie in Tabelle 1 ausgeführt gelöst und zusätzlich für 5 min bei 4°C mit einem Ultraschallbad (RK 100H, Bandelin, Germany) behandelt. Danach wurden alle Lösungen bei 4°C für die weiteren Schritte temperiert. 1-Ethyl-3-(3 dimethylaminopropyl)carbodiimid (EDC; Sigma-Aldrich, Deutschland) und N-hydroxysulfosuccinimide (sulpho-NHS; Sigma-Aldrich, Deutschland) wurden ebenfalls jeweils in einem Sechstel der gesamten Reaktionsmischung gelöst und bei 4°C temperiert. Das Molverhältnis von UGB1 : EDC : sulfoNHS beträgt 1:8:4 (1:2:1 für jede Aminogruppe des UGB1). Als nächster Schritt wurde das EDC und das sulfoNHS zum GB4 oder GB5 gegeben und durch Pipettieren vermischt und 30 Sekunden gewartet, im Anschluss die Reaktionsmischung 10s auf dem Vortex-Schüttler (VWR, Deutschland) vermischt und dann noch einmal 20 Sekunden gewartet. Nach dieser Prozedur von insgesamt 1 min Aktivierungszeit wurde der UGB1 auf dem Vortex-Schüttler durch Pipettieren zum aktivierten GB4 oder GB5 zugegeben und anschließend weitere 10s mit dem Vortex-Schüttler vermischt. Die fertige Reaktionsmischung (die Konzentrationen der UGB1 und GB4 oder GB5 entsprechend nunmehr den in der Tabelle 1 angegebenen Konzentrationen) konnte nun durch Pipettieren in beliebige Formen gegossen werden und die Gelbildung fand durch Polymerisation über einen Zeitraum von 12 Stunden statt. Im Anschluss wurden Die Hydrogele in phosphatgepufferter Kochsalzlösung durch mehrfachen Lösungsaustausch über mehrere Stunden komplett gequollen vor der weiteren Nutzung oder Charakterisierung der Hydrogele.

**Herstellung der Hydrogele für die Zellkultur mit humanen Endothelzellen:**

Für die Kultur humaner Endothelzellen wurden oberflächengebundene Gele mit einer finale Dicke von ca. 100 $\mu$m durch Pipettieren von 11 $\mu$l der fertigen Reaktionsmischung/cm2 auf Glasdeckgläsern geformt. Die Glasdeckgläser wurden hierfür zuvor mit einem Dünnfilm aus Poly(ethylene-alt-Maleinsäureanhydrid) gemäß der Prozedur von Pompe et. al. Biomacromolecules 2003, 4, 1072-1079 beschichtet, um eine kovalente Anbindung des Hydrogeles zu gewährleisten. Zur Modifikation mit RGD-Peptiden wurden die ausgeformten, in phosphatgepufferter Kochsalzlösung gequollenen Hydrogele mit EDC/sulfo-NHS mit einer Konzentration von 50 mM EDC und 25 mM sulpho-NHS in 1/15 M phosphatgepufferter Kochsalzlösung bei 4°C gelöst und noch verbleibende Carboxylgruppen der GB 4 oder GB5 für 20 min aktiviert und im Anschluss mit Boratpuffer (100 m M, pH 8.0, 4 ° C) gespült. Im Anschluss wurden die aktivierten Hydrogele mit einer Lösung einer Konzentration von 50 mg/mL der Peptidsequenz H2N-GWGG**RGD**SP-CONH2 (Peptides International, Louisville, KY, USA) gelöst in Boratpuffer (100 m M , pH 8.0) bei Raumtemperatur für 2 Stunden reagiert und im Anschluss exzessiv mit phosphatgepufferter Kochsalzlösung gewaschen.

**Zellkultur:**

Humane Nabelschnurvenenendothelzellen (HUVECs, Lonza, Deutschland) wurden bis zum Erreichen von 80% Konfluenz in Promocell C-22010 mit Zusatzmix (PromoCell GmbH, Germany) bei 37 °C und 5% CO2 auf mit Fibronektin beschichteten Zellkulturflaschen passagiert. Zellen der Passage 2 bis 6 wurden in den nachfolgenden Experimenten genutzt.

Die mit RGD-funktionalisierten oberflächengebundenen Hydrogele wurden mit VEGF-A und FGF-2 mit einer Konzentration von 0,565 $\mu$g je cm2 Hydrogelfläche für 18 Stunden bei RT inkubiert und dann 2x für mit phosphatgepufferter Kochsalzlösung gewaschen.

50.000 Zellen /cm2 wurden auf den mit RGD- und VEGF-165 und FGF-2-funktionalisierten Hydrogelen in Promocell medium ohne den Zusatzmix kultiviert. Nach 24 Stunden Kultur wurden die Zellen gewaschen, fixiert und mittels Lichtmikroskopie (Olympus IX73 invertiertes Miksroskop, Hamburg, Deutschland) charakterisiert. Das Aspektverhältnis wurde nach Ausmessen einzelner Zellen mittels Fiji-Bildbearbeitungssoftware aus der Division der Zelllänge durch die Zellbreite bestimmt. Die Zirkularität wurde mittels Fiji - Bildbearbeitungssoftware gemäß der folgenden Formel Zirkularität = 4*$\pi$*(Zellfläche/Perimeter)^2 bestimmt. Es wurden jeweils 20 Zellen je Bild auf n = 10 unabhängigen Proben bestimmt. Statistische Auswertung mit der GraphPad Prism Software und einem One-way-Anova-Test ergaben signifikante Unterschiede für alle untersuchten Bedingungen.

**Herstellung der Hydrogele basierend auf Poly(4-Styrensulfonsäure-co-maleinsäure) nach**

**Vernetzungsreaktion 2 (VN2, siehe auch Tabelle 1):**

**Derivatisierung von Poly(4-Styrensulfonsäure-co-maleinsäure) mit Maleimidgruppen:**

Für die Bildung von Hydrogelen gemäß der Vernetzungsreaktion 2 muss der GB4 oder GB5 zunächst mit N-(2-Aminoethyl)maleimid-trifluoracetat (Sigma, Deutschland) funktionalisiert werden. Hierfür wurden 500 mg (25 $\mu$mol) des jeweiligen GB4 oder GB5 in 2,7 ml deionisierten, ultrareinem Wasser gelöst und für 10 min auf Eis gerührt. Als Probengefäß dient ein 25 ml Schnappdeckelglas. Anschließend werden 65,14 mg NHS (0,30 mmol) gelöst in 400 $\mu$l und 115,02 mg (0,60mmol) EDC in gelöst in 200 $\mu$l eiskaltem deionisierten, ultrareinem Wasser der GB4 oder GB5-Lösung hinzugegeben und 5 min gewartet. Im nächsten Schritt wurden 76,25 mg (0,30 mmol) N-(-2-aminoethyl)-maleimid trifluoracetat gelöst in 200 $\mu$l MilliQ-Wasser nach der abgeschlossenen Aktivierungszeit dem aktiviertem GB4 oder GB5 tropfenweise über 40 s hinzugegeben. Das Reaktionsgemisch wird für weitere 10 min auf Eis gerührt. Zuletzt wird das Eisbad entfernt und der Ansatz über Nacht bei Raumtemperatur gerührt. Das Produkt wird in einen Dialyseschlauch mit einer Ausschlussgröße von MWCO=5 kDa gegeben. Die Dialyse erfolgt über zwei Tage, wobei am ersten Tag gegen 2,5 1 einmolare Natriumchlorid-Lösung für 6 h dialysiert wird. Hierbei wird die Lösung jeweils nach 2 h ausgetauscht. Nach 6 h wird anschließend über Nacht gegen deionisierten, ultrareinem Wasser dialysiert. Am zweiten Tag erfolgt die Dialyse für 8 h gegen deionisierten, ultrareinem Wasser, wobei nach jeweils 2 h das Wasser ausgetauscht wird. Im letzten Schritt wird die Lösung gefriergetrocknet.

**Größenausschlusschromatographie zur GB4/GB5-Maleimid-Derivate Charakterisierung:**

Die Größenausschlusschromatographie (SEC) wird zur Bestimmung der Anzahl an Maleimidgruppen je GB4 oder GB5-Molekül verwendet. Hierfür wird das Peptid RGD-SP (M=990 g/mol) in verschiedenen Überschüssen an das GB4/GB5-Maleimid-Derivate gekoppelt. Die untersuchten molaren RGD-SP-Überschüsse betragen hierbei 6,8, 10 bzw. 12 gegenüber dem GB4 oder GB5. Zunächst wird eine Kalibrierung aufgenommen indem 35 $\mu$l der jeweiligen RGD-SP-Konzentration mit 35 $\mu$l phosphatgepufferter Kochsalzlösung in einem Probengefäß vermischt werden.

Für diese Untersuchung wird eine BioSEP-SEC S2000-Säule von Phenomnex (Deutschland) verwendet, die in das HPLC-System Agilent 1100 (Deutschland) eingesetzt ist. Als Eluent dient phosphatgepufferter Kochsalzlösung mit einer Durchflussrate von 0,5 ml/min. Das Injektionsvolumen beträgt 50 $\mu$l.

Bei gleichen Elutionsbedingungen wird auch die Bestimmung der Maleimidgruppen durchgeführt. Hierbei werden jeweils 35 $\mu$l einer RGD-Konzentration mit 35 $\mu$l der GB4/GB5-Maleimid-Derivate Lösung im Probengefäß vermischt. Die Methode erlaubt die exakte Bestimmung der Umsätze des Maleimid mit den GB4 oder GB5.

**Herstellung der Hydrogele basierend auf Poly(4-Styrensulfonsäure-co-maleinsäure)-Derivaten nach Vernetzungsreaktion 2 (VN2, siehe auch Tabelle1):**

GB4/GB5-Maleimid-Derivate und vierarmiges, thiolterminiertes PEG (UGB2, Mw=10.000 Da, Jenchem, USA, Tabelle 2) werden in phosphatgepufferter Kochsalzlösung mit der zweifachen Konzentrationen wie in Tabelle 1 genannt gelöst. Danach werden gleiche Volumen beider Komponenten in ein Mikroreaktionsgefäß pipettiert und 10s auf dem Vortex-Schüttler geschüttelt. Im Anschluss werden ein definiertes Volumen des Reaktionsgemisches entnommen und auf ein mit Sigma Cote® beschichtetes Deckgläschen, mit 9 mm Durchmesser gegeben. Ein weiteres mit Sigma Cote® beschichtetes Deckgläschen wird auf den Tropfen platziert. Nach 30 min werden die Deckgläschen entfernt und die Gelscheibe über 12 Stunden in phosphatgepufferter Kochsalzlösung gequollen. Bei hohen UGB2-Konzentrationen kann der pH-Wert des UGB2 auf einen Wert zwischen pH 5-7 mit 1 M HCl angepasst werden, um eine optimale Gelbildung zu erreichen.

**Herstellung der Hydrogele für die Zellkultur mit humanen mesenchymalen Stromazellen in 3D:**

Matrix-Metalloprotease (MMP) spaltbare Hydrogele wurden präpariert durch Nutzung des UGB4 (Tabelle 4, synthetisiert nach der Prozedur beschrieben bei Tsurkan et al., Adv. Mater. 2013, 25 (18), 2606-2610) anstelle des UGB2 nach der zuvor genannten Methode.

Die Zellen wurden hierfür im GB4 oder GB5-Derivat suspendiert und im Anschluss entsprechend der oben genannten Prozedur die Hydrogele durch Mischung geformt und direkt nach 5 min Gelierungszeit in phosphatgepufferter Kochsalzlösung und Zellkulturmedium gequollen.

**Zellkultur:**

Mesenchymale Stromazellen (MSCs, ATCC, isoliert aus Fettgewebe, Deutschland) wurden in DMEM mit 10% fötalem Kälberserum und 1% Penicillin/Streptomyzin unter Standardkulturbedingungen (5% $CO_2$ und 37°C) kultiviert. MSCs der Passagen 2 bis 4 wurden in den Experimenten genutzt.

GB-4 oder GB-5-Derivate und UGBs wurden mit 1 mol CGWGGRGDSP pro mol GB vermischt und in phosphatgepufferter Kochsalzlösung aufgelöst (die Konzentration der GB4 oder GB5 Derivate entsprach der 3fachen Konzentration entsprechend der Tabelle 1). Die Lösung wurden nach der Mischung für 10-15 min bei 37°C inkubiert und anschließend eine konzentrierte Zellsuspension in einem Drittel des finalen Gelvolumens mit $6 \times 10^6$ Zellen /ml hochgerechnet auf die finale Reaktionsmischung vermischt. Das UGB4 (3 fache Konzentration der Tabelle 1) wurde in einem weiteren drittel der gesamten Gelvolumen in phosphatgepufferter Kochsalzlösung aufgelöst und zur Mischung aus GB4/5-Derivaten mit Zellen gegeben und durch Pipettieren vermischt. Nach der Gelbildung für 5 min wurde das oben beschriebene Zellkulturmedium zugegeben und die Hydrogele wie zuvor beschrieben für 24h bei 37°C inkubiert. PrestoBlue®-Test: Der PrestoBlue®-Test wird nach 24 h Inkubationszeit nach der Kultur der Zellen in den Hydrogelen durchgeführt. Hierfür werden 10%ige Lösung des PrestoBlue®-Farbstoffes gelöst in Zellkulturmedium zu jeder Hydrogelprobe hinzugegeben und in Mikrotiterplatten für 1 h bei 37°C inkubiert. Anschließend erfolgt die Messung der Fluoreszenz am Mikroplatten-Photometer (Tecan Spark®, Deutschland). Die Anregungswellenlänge beträgt 560 nm und die Messung der Fluoreszenz erfolgte bei einer Wellenlänge von 590 nm. Die metabolische Aktivität der Zellen wurde anhand der relativen Fluoreszenzwerte (RFUs) angegeben. Es wurden 10 unabhängige Versuche mit 4 Fachbestimmung (n = 40) ausgewertet und signifikante Unterschiede (One Way Anova) für alle Bedingungen gefunden.

**Antibakterieller Inhibierungszonenassay:**

Hydrogel Disks (60 µl) wurden in 1 ml von 50 µg/ml Gentamiycin (Sigma Aldrich, Germany) für 18 Stunden inkubiert und dann zweifach mit phosphatgepufferter Kochsalzlösung gewaschen. Die antimikrobielle Aktivität der mit Gentamiycin beladenen Hydrogele wurde anhand eines Inhibitionszonen-Test bestimmt. Hierfür wurde Luria broth (LB) agar (Sigma Aldrich, Munich, Germany) Platten entsprechend der Herstellerangaben präpariert. Escherichia coli K12 DH5 (DSMZ, Germany) and Staphylococcus epidermidis PCI 1200 (ATCC, USA) Kulturen nach 12h Wachstum wurden zu einer optischen Dichte (OD) gemessen bei 600 nm von 0,1 verdünnt und 250 µl dieser Bakteriensuspension wurden auf die jeweiligen Platten gegeben. Ein steriles Baumwolltüchlein wurde zur gleichmäßigen Verteilung der Bakterien genutzt. Hydrogele UGB1-GB1 01, UGB2-GB4 20 und UGB2-GB5 23 sowie UGB2-UGB3 26 jeweils beladen oder unbeladen mit Gentamicin wurden in den vier Ecken auf der AGAR-Platte platziert und die Platten übernacht bei 37°C inkubiert. Danach wurde die Inhibierungszone mittels eines digitalen Messschiebers vermessen. Es wurden 10 unabhängige Versuche mit 3 Fachbestimmung (n = 30) ausgewertet und signifikante Unterschiede (One Way Anova) für alle Bedingungen gefunden.

**Bestimmung der physikalisch-chemischen Eigenschaften der Hydrogele:**

Zur Bestimmung der physikalischen Eigenschaften der verschiedenen Hydrogeltypen wurden jeweils 67 µl unpolymerisierte Hydrogellösung zwischen zwei mit Sigmacote (Sigma-Aldrich, Deutschland) behandelten 9 mm Glasträgern (Menzel Gläser, Deutschland) für 16 h bei Raumtemperatur auspolymerisiert und anschließend die resultierenden Gelscheiben von den Glasträgern entfernt. Der Durchmesser der Gelscheiben wurde mittels eines Scanner des Typs FLA-3100 (Fujitsu, Japan) optisch bestimmt (Durchmesser im ungequollenen Zustand). Im Anschluss wurden die Gelscheiben in phosphat-gepufferter Kochsalzlösung (PBS), 0,9 % NaCl gepuffert auf pH 7,4 (Sigma-Aldrich, Deutschland) für 24 h gequollen (physiologische Bedingungen) und erneut mittels des Scanner des Typs FLA-3100 (Fujitsu, Japan) bestimmt (Durchmesser im gequollenen Zustand). Die Quellung der Hydrogele wurde aus den bestimmten Durchmessern nach folgender Gleichung bestimmt:

Quellung = Durchmesser des gequollenen Hydrogels^3 / Durchmesser des ungequollenen Hydrogels^3,

$$Quellung = \frac{Durchmesser\ des\ gequollenen\ Hydrogels^3}{Durchmesser\ des\ ungequollenen\ Hydrogels^3}$$

siehe Tabelle 1.

Die berichteten Daten sind durch Mittelwertbildung (MW) aus jeweils mindestens vier unabhängigen Proben erhalten. Zusätzlich wurde die Standardabweichung (SD) angegeben.

Weiterhin wurde das Speichermodul und das Verlustmodul der Hydrogele mittels oszilatorischer Rheometrie (in Kilopascal) mittel eines Scherrheometers des Typs Ares der Firma TA Instruments United Kingdom, bestimmt. Dazu wurden 8 mm Scheiben aus unter physiologischen Bedingungen (phosphatgepufferte Kochsalzlösung (PBS, 0,9 % NaCl gepuffert auf pH 7,4 (Sigma-Aldrich, Deutschland)) für 24 h gequollenen Hydrogelen ausgestanzt und in einer 9 mm Platte-Platte Messanordnung mit steigender Frequenz von 1-100 rad/s bei Raumtemperatur bei geringer Deformation (2%) gemessen und der Mittelwert über den gesamten Frequenzbereich bestimmt (1 Messwert an einer Probe). Die berichteten Werte sind die Mittelwerte von vier unabhängigen voneinander hergestellten Hydrogeldisks und werden +/- der Standardabweichung (SD) angegeben. Das Speichermodul der vier Hydrogeltypen wurde in Tabelle 1 angegeben, das Verlustmodul war jeweils um mehrerer Größenordnungen geringer (Daten nicht gezeigt).

In Tabelle 1 sind die physikochemischen Eigenschaften der Hydrogele aufgezeigt.

Die Maschenweite der Hydrogele kann auf Basis der Gummielastizitätstheorie von den experimentell bestimmten Speichermodul mit Hilfe folgender Formel abgeleitet werden (Polymer Physics, Michael Rubinstein and Ralph H. Colby, 2006, Oxford University Press, Oxford):

$$\xi = \left(\frac{G'N_A}{RT}\right)^{-1/3}$$

Wobei G' das gemessene Speichermodul, $N_A$ die Avogadro-Konstante, R die universelle Gaskonstante und T die Temperatur (in Kelvin) ist.

Zur Bestimmung der Bindungseigenschaften der unterschiedlichen Hydrogeltypen wurden unpolymerisierte Hydrogellösungen zwischen zwei mit Sigmacote (Sigma-Aldrich, Deutschland) behandelten 5 mm Glasträgern (Menzel Gläser) für 16 h bei Raumtemperatur auspolymerisiert und anschließen in phosphat-gepufferter Kochsalzlösung (PBS), 0,9 % NaCl gepuffert auf pH 7,4 (Sigma-Aldrich, Deutschland) für 24 h gequollen. Das Gesamtvolumen wurde dabei entsprechend angepasst, um gleiche molare Mengen des GB1, GB2, GB3, und GB4 in den vier Hydrogeltypen zu gewährleisten (siehe Tabelle 4). Für die Bindungs (Sequestrierungs) Studien wurden die jeweiligen Hydrogelscheiben für 24 h in 0.5 ml Protein LoBind Reaktionsgefäßen (Eppendorf Tubes, Germany) mit dem Proteingemisch entsprechend (Tabelle 3) gelöst in 400 µl PBS mit 1% (m/v) bovinen Serumalbumin (BSA; Sigma-Aldrich, Deutschland) und 0.05% (m/v) Proclin 300 (Sigma-Aldrich, Deutschland) inkubiert (entspricht Lösung nach der Inkubation 2). Zur Herstellung des Proteingemisches wurde die ProcartaPlex Standard A, B und C (ebioscience, Germany) entsprechend der Herstellerangaben gelöst und mit den Proteinen DKK1 (Herstellernr.: 120-30, Peprotech, Germany), Sclerostin (Herstellernr.: 1406-ST, Peprotech, Germany) und TGFbl (Herstellernr.: 100-21, Peprotech, Germany) supplementiert. Die Einzelkonzentrationen sind in Tabelle 7 dargestellt (entspricht Lösung vor der Inkubation 1).

Die Lösung 1 und 2 wurden bis zur Messung der Proteinkonzentration bei -80°C gelagert. Zur Bestimmung der Proteinkonzentrationen in den Lösungen 1 und 2 wurde die Proben nach Herstellerangaben mittels ProcartaPlex Human Chemokine Panel 1 (ebioscience, Deutschland) in Kombination mit den entsprechenden ProcarteaPlex Simplex Kits auf einen Gerät des Typs Bioplex 200 (Biorad, Deutschland) gemessen.

Die Bindung (Sequestrierung) der Signalmoleküle wurde aus den ermittelten Konzentrationen der Lösung 1 und 2 nach folgender Gleichung bestimmt: Bindung in % = (1 - Konzentration in Lösung 2 (nach der Inkubation)/Konzentration in Lösung 1 (vor Inkubation)) x 100.

In Tabelle 4 ist die Aktivierungszeit der Carboxylgruppen der GB 1 bis 4 in min und das genutzte Gelvolumen für die Bindungsstudien tabellarisch dargestellt.

Die sulfatierten bzw. sulfonierten Hydrogele sind durch ihre Ladungsverteilung an dem geladenen Baustein (GB) in mol Sulfat- bzw. Sulfonatgruppen je mol Polymer sowie der Konzentration an Sulfat- bzw. Sulfonatgruppen im unter physiologischen Bedingungen gequollenen Hydrogelvolumen in mmol Sulfat bzw. Sulfonat/ml Hydogel sowie der Anzahl an Sulfat- bzw. Sulfonatgruppen je WE geteilt durch die Molmasse der WE in den angegebenen Bereichen charakterisiert. Die Berechnung erfolgte aus den molaren Konzentrationen der Hydrogelbausteine bei der Hydrogelbildung (siehe Tabelle 1) und der Volumenquellung, Tabelle 1) unter der Annahme, dass die Hydrogelbausteine quantitativ in das Netzwerk eingebaut werden.

Die Konzentration der Sulfat oder Sulfonatgruppen im ungequollenen Hydrogel wurde aus der Konzentration der GBs im ungequollenen Hydrogel x Anzahl der Wiederholeinheiten x Nummer der Sulfat oder Sulfonatgruppen je Wiederholeinheit berechnet. Die Konzentration der Sulfat oder Sulfonat im gequollenen Gel (Tabelle 1) wurde aus der Konzentration der Sulfat oder Sulfonatgruppen im ungequollenen Hydrogel geteilt durch die Quellung berechnet (siehe Tabelle 1).

Extraktionsversuche haben keine messbare Eluation der Gelbausteine ergeben, so dass die Annahme des vollständigen Einbaus der Gelbausteine als gerechtfertigt angesehen wird.

In Tabelle 3 sind die prozentual gebundenen Mengen an Signalmolekülen in den Hydrogelen normalisiert zur Konzentration der löslichen Faktoren vor der Inkubation angegeben, wie vorangehend erläutert. Die Prozentangaben beziehen sich auf Masseprozent.

Die molekulare Größe der Stoffe, die auch als Signalstoffe oder Faktoren bezeichnet werden, wird in Kilo-Dalton (kDa) angegeben.

Die Proteine (Signalmoleküle) sind in Abkürzungen (Tabelle 3 und den UniProt Identifikationsnummern (Uniprot ID) der Proteindatenbank Universal Protein Ressource (UniProt; http://www.uniprot.org/) eindeutig zugeordnet.

Der isoelektrische Punkt (IEP) und die Molmasse wurde aufgrund der vollständig biologisch prozessierten Aminosäuresequenz mittels dem Programm ExPASy ProtParm (http://web.expasy.org/protparam/; Referenz: Gasteiger, E. et al. in The Proteomics Protocols Handbook 571-607 (2005)) berechnet.

Die Strukturparameter der Proteine wurde mittels der Datenbank ExPASy PROSITE auf Basis der UniProt Identifikationsnummern bestimmt (http://prosite.expasy.org/; Sigrist, C. J. A. et al. New and continuing developments at ExPASy PROSITE; Referenzen: (1) Nucleic Acids Res. 41, (2013); (2) Sigrist, C. J. a et al. PROSITE: a documented database using patterns and profiles as motif descriptors. Brief. Bioinform. 3, 265-274 (2002)).

**Patentansprüche**

1. Verfahren zur differenzierten Sequestrierung von Stoffen verschiedener Stoffgruppen A und B in einem sulfatierten und/oder sulfonierten Hydrogel und Abreicherung der Stoffe der Stoffgruppe A aus einem Biofluid bei gleichzeitiger differenzierter Freisetzung von Stoffen der Stoffgruppe A oder B aus dem sulfatierten und/oder sulfonierten Hydrogel in das Biofluid oder der reduzierten Bindung der Stoffe der Stoffgruppe B im sulfatierten und/oder sulfonierten Hydrogel, wobei das sulfatierte und/oder sulfonierte Hydrogel aus einer Gruppe von Hydrogelen vom Typl, Typ2, Typ3 oder Typ4 ausgewählt ist und die Hydrogele aus ungeladenen Bausteinen und geladenen Bausteinen bestehen welche **dadurch gekennzeichnet sind, dass** die geladenen Bausteine durch einen Parameter berechnet aus der Anzahl an Sulfat- und/oder Sulfonatgruppen je Wiederholeinheit geteilt durch die Molmasse der Wiederholeinheit für Typ1 von 0,0040-0,0060 mol/g, für Typ2 0,0025-0,0040 mol/g, für Typ3 0,0005-0,0025 mol/g und für Typ4 0,0040-0,0100 mol/g charakterisiert sind und die gequollenen Hydrogele ein Speichermodul von kleiner als 20 kPa aufweisen und die Eigenschaften der gequollenen Hydrogele eine Konzentration an Sulfat- oder Sulfonatgruppen in mmol/ml bei Typ1 von 0,09 bis 0,20, bei Typ2 von 0,05 bis 0,18, bei Typ3 von 0,01 bis 0,12 und bei Typ4 von 0,16 bis 0,8 aufweisen und dass eine Beeinflussung der Konzentration von Stoffen der Stoffgruppe A und von Stoffen der Stoffgruppe B im Biofluid durch die Wahl des Typs der Hydrogele erfolgt.

2. Verfahren nach Anspruch 1, **dadurch gekennzeichnet, dass** die geladenen Bausteine der Hydrogele eine Anzahl an Sulfat- und/oder Sulfonatgruppen je Wiederholeinheit geteilt durch die Molmasse der Wiederholeinheit für Typ1 von 0,0050 mol/g, für Typ2 0,0035 mol/g oder 0,0038 mol/g, für Typ3 0,0019 mol/g und für Typ4 0,0045 mol/g aufweisen und die Eigenschaften der gequollenen Hydrogele eine Konzentration Sulfat oder Sulfonat in mmol/ml bei Typ1 von 0,12 und bei Typ2 von 0,12 oder 0,14 und bei Typ3 von 0,06 und bei Typ4 von 0,28 oder 0,16 aufweisen.

3. Verfahren zur differenzierten Sequestrierung von Stoffen verschiedener Stoffgruppen A und B in einem sulfatierten und/oder sulfonierten Hydrogel und Abreicherung der Stoffe der Stoffgruppe A aus einem Biofluid bei gleichzeitiger differenzierter Freisetzung von Stoffen der Stoffgruppe B aus dem sulfatierten und/oder sulfonierten Hydrogel in das Biofluid oder der reduzierten Bindung der Stoffe der Stoffgruppe B im sulfatierten und/oder sulfonierten Hydrogel, wobei das sulfatierte und/oder sulfonierte Hydrogel aus einer Gruppe von Hydrogelen vom Typl, Typ2, Typ3 oder Typ4 ausgewählt ist und die Hydrogele aus ungeladenen Bausteinen und geladenen Bausteinen bestehen welche **dadurch gekennzeichnet sind, dass** die geladenen Bausteine eine Konzentration von mol Sulfat- oder Sulfonatgruppen pro mol Polymer bei Typ1 von 60 bis 80, bei Typ2 von 30 bis 75, bei Typ3 von 10 bis 30 und bei Typ4 von 80 bis 120 aufweisen und die geladenen Bausteine im Hydrogel eine Konzentration GB in mmol/ml bei Typ1 von 0,0015 bis 0,0025 bei Typ2 von 0,0015 bis 0,0030, bei Typ3 von 0,0010 bis 0,0040 und bei Typ4 von 0,0018 bis 0,0050 aufweisen und die gequollenen Hydrogele ein Speichermodul von kleiner als 20 kPa aufweisen, wobei die Sulfat- oder Sulfonatgruppen eine Konzentration in mmol pro ml im Hydrogel bei Typ1 von 0,09 bis 0,20, bei Typ2 von 0,05 bis 0,18, bei Typ3 von 0,01 bis 0,12 und bei Typ4 von 0,16 bis 0,80 aufweisen und dass eine Beeinflussung der Konzentration von Stoffen der Stoffgruppe A und von Stoffen der Stoffgruppe B im Biofluid durch die Wahl des Typs der Hydrogele erfolgt.

4. Verfahren nach Anspruch 3, **dadurch gekennzeichnet, dass** die geladenen Bausteine eine Konzentration von mol Sulfat- oder Sulfonatgruppen pro mol Polymer bei Typ1 von 70,2 und bei Typ2 von 48,4 oder 75,0 und bei Typ3 von 23,4 und bei Typ4 von 90,0 aufweisen und die geladenen Bausteine im Hydrogel eine Konzentration GB in mmol/ml bei Typ1 von 0,0018, bei Typ2 von 0,0025 oder 0,0018, bei Typ3 von 0,0027 und bei Typ4 von 0,0031oder 0,0018 aufweisen und die gequollenen Hydrogele ein Speichermodul von kleiner als 20 kPa aufweisen, wobei die Konzentration von Sulfat- oder Sulfonatgruppen in mmol pro ml im Hydrogel bei Typ1 von 0,12, bei Typ2 von 0,12 oder 0,14, bei Typ3 von 0,06 und bei Typ4 von 0,28 oder 0,16 beträgt.

5. Verfahren nach einem der Ansprüche 1 bis 4, **dadurch gekennzeichnet, dass** die Vernetzung der Bausteine zum Hydrogel über Reaktion geeigneter funktioneller Gruppen an den GB und/oder den UGB aus der Klasse der Amine, Thiole, Carboxyle, Anhydride, Maleimide, Vinylsulfone, Acrylate, Hydroxyle, Isocyanate, Epoxide und Aldehyde oder Gruppen, die nichtkovalente Bindungen basierend auf elektrostatischen Kräften oder hydrophoben Wechselwirkungen, Wasserstoffbrückenbindungen oder Dipolwechselwirkungen ausbilden können, erfolgt.

6. Verfahren nach einem der Ansprüche 1 bis 5, **dadurch gekennzeichnet, dass** die Vernetzung der geladenen Bausteine zum Hydrogel über direkte Vernetzung mittels eines kleinen bifunktionellen Vernetzungsmolekül mit einer Molmasse UGB< 500 g/mol erfolgt.

7. Verfahren nach einem der Ansprüche 1 bis 6, **dadurch gekennzeichnet, dass** als geladenen Bausteine aus natürlichen Quellen gewonnene sulfatierte Glykosaminoglykane, wie Heparin und selektive desulfatierte Heparine, Chondroitinsulfat, Heparansulfat, Keratansulfat, sulfatierte Hyaluronsäure, ebenso sulfatierte Glycopolymere auf Basis von Mannose, Lactose, Dextran sowie polysulfonierte Verbindungen, welche Styrensulfonsäure (SS), Vinylsulfonsäure (VS), 2-Acrylamido-2-methylpropansulfonsäure (AMPS), Aminopropansulfonsäure (APS) oder Anetholesulfonsäure (AS) als schwefelhaltige Monomere tragen und auch in Copolymeren mit Einheiten, die die zuvor genannten Vemetzungsgruppen enthalten ausgewählt sind und dass als ungeladenen Bausteine Polyethylenglykole, Poly(2-oxazoline), Polyvinylpyrrolidone (PVP), Polyvinylalkohole (PVA), und/oder Polyacrylamide (PAM) oder eines kurzen bifunktionellen Vernetzermolekül ausgewählt sind.

8. Verfahren nach einem der Ansprüche 1 bis 7, **dadurch gekennzeichnet, dass** bei Hydrogelen vom Typ1 als Stoffe der Stoffgruppe A mindestens einer der Stoffe bNGF, PDGF-BB, VEGF-A, Eotaxin, GRO-alpha, IL-8, IP-10, MCP-1, MIP-1 alpha, MIP-1 beta, Rantes, SDF1-alpha, IFN-gamma, IL-12p40, IL-4, Sclerostin und/oder DKK1 ausgewählt ist, wobei die Stoffe zu mehr als 50% der initialen Konzentration der Lösung, aus dem Biofluid abgereichert und im Hydrogel sequestriert werden.

9. Verfahren nach einem der Ansprüche 1 bis 8, **dadurch gekennzeichnet, dass** bei Hydrogelen vom Typ1 als Stoffe der Stoffgruppe B mindestens einer der Stoffe FGF-2, TGFbl, EGF, HGF, PLGF, GM-CSF, IL-1 beta, IL-10, IL-6 und/oder TNF-alpha ausgewählt ist, wobei die Stoffe nur bis zu 50% der initialen Konzentration der Lösung im Hydrogel gebunden oder aus diesem freigesetzt werden.

10. Verfahren nach einem der Ansprüche 1 bis 9, **dadurch gekennzeichnet, dass** bei Hydrogelen vom Typ2 als Stoffe der Stoffgruppe A mindestens einer der Stoffe bNGF, PDGF-BB, VEGF-A, Eotaxin, GRO-alpha, IL-8, IP-10, MCP-1, Rantes, SDF1-alpha, IFN-gamma, IL-12p40, IL-4 und/oder DKK1 ausgewählt ist, wobei die Stoffe zu mehr als 50% der initialen Konzentration der Lösung aus dem Biofluid abgereichert und im Hydrogel sequestriert werden.

11. Verfahren nach einem der Ansprüche 1 bis 10, **dadurch gekennzeichnet, dass** bei Hydrogelen vom Typ2 als Stoffe der Stoffgruppe B mindestens einer der Stoffe FGF-2, TGFbl, EGF, HGF, PLGF, GM-CSF, IL-1 beta, IL-10, IL-6, TNF-alpha und/oder Sclerostin ausgewählt ist, wobei die Stoffe nur bis zu 50% der initialen Konzentration der Lösung im Hydrogel gebunden oder aus diesem freigesetzt werden.

12. Verfahren nach einem der Ansprüche 1 bis 11, **dadurch gekennzeichnet, dass** bei Hydrogelen vom Typ3 als Stoffe der Stoffgruppe A mindestens einer der Stoffe bNGF, PDGF-BB, VEGF-A, Eotaxin, GRO-alpha, IP-10, Rantes, SDF1-alpha, IFN-gamma und/oder IL-4 ausgewählt ist, wobei die Stoffe zu mehr als 50% der initialen Konzentration der Lösung aus dem Biofluid abgereichert und im Hydrogel sequestriert werden.

13. Verfahren nach einem der Ansprüche 1 bis 12, **dadurch gekennzeichnet, dass** bei Hydrogelen vom Typ3 als Stoffe der Stoffgruppe B mindestens einer der Stoffe FGF-2, TGFbl, EGF, HGF, PLGF, IL-8, MCP-1, MIP-1 alpha, MIP-1 beta, GM-CSF, IL-1 beta, IL-10, IL-12p40, IL-6, TNF-alpha, Sclerostin und/oder DKK1 ausgewählt ist, wobei die Stoffe nur bis zu 50% der initialen Konzentration der Lösung im Hydrogel gebunden oder aus diesem freigesetzt werden.

14. Verfahren nach einem der Ansprüche 1 bis 13, **dadurch gekennzeichnet, dass** bei Hydrogelen vom Typ4 als Stoffe der Stoffgruppe A mindestens einer der Stoffe bNGF, PDGF-BB, VEGF-A, Eotaxin, GRO-alpha, IL-8, IP-10, MCP-1, MIP-1 alpha, MIP-1 beta, Rantes, SDF1-alpha, IFN-gamma, IL-1 beta, IL-10, IL-12p40, IL-4, IL-6, und/oder TNF-alpha ausgewählt ist, wobei die Stoffe zu mehr als 60 % der initialen Konzentration der Lösung aus dem Biofluid abgereichert und im Hydrogel sequestriert werden.

15. Verfahren nach einem der Ansprüche 1 bis 14, **dadurch gekennzeichnet, dass** bei Hydrogelen vom Typ4 als Stoffe der Stoffgruppe B mindestens einer der Stoffe EGF, PLGF und/oder GM-CSF ausgewählt ist, wobei die Stoffe nur bis zu 40% der initialen Konzentration der Lösung im Hydrogel gebunden oder aus diesem freigesetzt werden.

16. Verfahren nach einem der Ansprüche 1 bis 15, **dadurch gekennzeichnet, dass** Hydrogele vom Typ 1, Typ 2, Typ 3 oder Typ 4 mit mindestens einem Stoff der Stoffgruppe A und/oder B in hoher Konzentration vorbeladen und dann diese Stoffe parallel und unabhängig zur Konzentrationsveränderung im Biofluid aus dem Hydrogel freigesetzt werden.

17. Verfahren nach einem der Ansprüche 1 bis 16, **dadurch gekennzeichnet, dass** die Hydrogele aus mehrphasigen Materialien ausgebildet sind, d.h. aus Mischungen der unterschiedlichen Typen 1-4 der Hydrogelmaterialien bzw. unterschiedlicher Verarbeitungsformen (Mikropartikel und Bulkhydrogele) bzw. mit unterschiedlichen Stoffen oder Stoffmengen vorbeladen sind.

18. Verfahren nach einem der Ansprüche 1 bis 17, **dadurch gekennzeichnet, dass** die differenzierte Freisetzung von Stoffen der Stoffgruppe A oder B aus dem sulfatierten und/oder sulfonierten Hydrogel in das Biofluid und die reduzierte Bindung der Stoffe der Stoffgruppe B im sulfatierten und/oder sulfonierten Hydrogel gleichzeitig erfolgen.

## Claims

1. A method for differentiated sequestration of substances of different substance groups A and B in a sulfated and/or sulfonated hydrogel and for depletion of substances of substance group A from a biofluid with simultaneous differentiated release of substances of substance group A or B from the sulfated and/or sulfonated hydrogel into the biofluid or the reduced binding of substances of the substance group B in the sulfated and/or sulfonated hydrogel, wherein the sulfated and/or sulfonated hydrogel is selected from a group of type 1, type 2, type 3 or type 4 hydrogels and the hydrogels are composed of uncharged building blocks and charged building blocks which **are characterized in that** the charged building blocks are **characterized by** a parameter calculated from the number of sulfate and/or sulfonate group per repeat unit divided by the molar mass of the repeat unit of 0.0040-0.0060 mole/g for type 1, of 0.0025-0.0040 mole/g for type 2, of 0.0005-0.0025 mole/g for type 3, and of 0040 to 0.0100 mole/g for type 4 and the swollen hydrogels have a storage module of less than 20 kPa and the properties of the swollen hydrogels have a concentration of sulfate or sulfonate groups in mmole/ml between 0.09 to 0.20 in type 1, between 0.05 to 0.18 for type 2, between 0.01 to 0.12 for type 3, and between 0.16 to 0.8 for type 4, and that an influence on the concentration of substances of substance group A and of substances of substance group B in the biofluid is determined by the choice of the type of the hydrogel.

2. The method according to claim 1, **characterized in that** the charged building blocks of the hydrogels have a number of sulfate and/or sulfonate groups per repeat unit divided by the molar mass of the repeat unit of 0.0050 mole/g for type 1, of 0.0035 mole/g or 0.0038 mole/g for Type 2, of 0.0019 mole/g for type 3, and of 0.0045 mole/g for type 4, and that the properties of the swollen hydrogels have a concentration of sulfate or sulfonate in mmole/ml of 0.12 for type 1 and of 0.12 or 0.14 for type 2 and of 0.06 for type 3 and of 0.28 or 0.16 for type 4.

3. A method for the differentiated sequestration of substances of different substance groups A and B in a sulfated and/or sulfonated hydrogel and depletion of substances of substance group A from a biofluid with simultaneous differentiated release of substances of substance group B from the sulfated and/or sulfonated hydrogel in the biofluid or the reduced binding of substances of substance group B in the sulfated and/or sulfonated hydrogel, wherein the sulfated and/or sulfonated hydrogel is selected from a group of hydrogels of type 1, type 2, type 3 or type 4 and the hydrogels are composed of uncharged building blocks and charged building blocks which **are characterized in that** the charged building blocks have a concentration of mole sulfate or sulfonate groups per mole of polymer of 60 to 80 in type 1, of 30 to 75 in type 2, of 10 to 30 in type 3 and of 80 to 120 in type 4, and that the charged building blocks in the hydrogel have a concentration GB in mmole/ml of 0.0015 to 0.0025 in type1, of 0.0015 to 0.0030 in type 2, of 0.0010 to 0.0040 in type 3, and of 0.0018 to 0.0050 in type 4, and that the swollen hydrogels have a storage modulus smaller than 20 kPa, with the concentration of the sulfate or sulfonate groups in mmole per ml in the hydrogel of type 1 between 0.09 and 0.20, in type 2 between 0.05 and 0.18, in type 3 between 0.01 and 0.12 and in type 4 between 0.16 and 0.80, and that the concentration of substances of substance group A and of substances of substance group B in the biofluid is influenced by the choice of the type of hydrogels.

4. The method according to claim 3, **characterized in that** the charged building blocks have a concentration of mole sulfate or sulfonate groups per mole of polymer of 70.2 in type 1 and of 48.4 or 75.0 in type 2 and of 23.4 in type 3 and of 90.0 in type 4, and that the charged building blocks in the hydrogel have a concentration GB in mmole/ml of 0.0018 for type 1, of 0.0025 or 0.0018 for type 2, of 0.0027 for type 3, and of 0.0031 or 0.0018 for type 4, and that the swollen hydrogels have a storage modulus of less than 20 kPa, with the concentration of sulfate or sulfonate groups in mmole per ml in the hydrogel being 0.12 for type 1, 0.12 or 0.14 for type 2, 0.06 for type 3 and 0.28 or 0.16 for type 4.

5. The method according to one of claims 1 to 4, **characterized in that** the crosslinking of the building blocks to the hydrogel takes place by reaction of suitable functional groups on the GB and/or UGB from the class of amines, thiols, carboxyls, anhydrides, maleimides, vinylsulfones, acrylates, hydroxyls, isocyanates, epoxides and aldehydes, or groups capable of forming noncovalent bonds based on electrostatic forces or hydrophobic interactions, hydrogen bonds or dipole interactions.

6. The method according to one of claims 1 to 5, **characterized in that** the crosslinking of the charged building blocks to the hydrogel takes place via direct crosslinking by way of a small bifunctional crosslinking molecule having a molar mass UGB <500 g/mole.

7. The method according to one of claims 1 to 6, **characterized in that** sulfated glycosaminoglycans obtained from natural sources, such as heparin and selective desulfated heparins, chondroitin sulfate, heparan sulfate, keratan sulfate, sulfated hyaluronic acid, as well as sulfated glycopolymers based on mannose, lactose, dextran and polysulfonated compounds which carry styrenesulfonic acid (SS), vinylsulfonic acid (VS), 2-acrylamido-2-methylpropanesulfonic acid (AMPS), aminopropanesulfonic acid (APS) or anetholesulfonic acid (AS) as sulfur-containing monomers and also in copolymers with units containing the aforementioned linking groups are selected as charged building blocks, and that polyethylene glycols, poly (2-oxazolines), polyvinylpyrrolidone (PVP), polyvinyl alcohols (PVA), and/or polyacrylamides (PAM) or a short bifunctional crosslinker molecule are selected as uncharged building blocks .

8. The method according to one of claims 1 to 7, **characterized in that** in hydrogels of type 1 at least one of the substances bNGF, PDGF-BB, VEGF-A, eotaxin, GRO-alpha, IL-8, IP-10 , MCP-1, MIP-1 alpha, MIP-1 beta, Rantes, SDF1-alpha, IFN-gamma, IL-12p40, IL-4, sclerostin and/or DKK1 is selected as substance of the substance group A, with the substances being depleted to more than 50% of the initial concentration of the solution from the biofluid and sequestered in the hydrogel.

9. The method according to one of claims 1 to 8, **characterized in that** in hydrogels of type 1 at least one of the substances FGF-2, TGFb1, EGF, HGF, PLGF, GM-CSF, IL-1 beta, IL-10, IL-6 and/or TNF-alpha is selected as substance of the substance group B, wherein the substances are bound only up to 50% of the initial concentration of the solution in the hydrogel or released therefrom.

10. The method according to one of claims 1 to 9, **characterized in that** in hydrogels of type 2 at least one of the substances bNGF, PDGF-BB, VEGF-A, eotaxin, GRO-alpha, IL-8, IP-10, MCP-1, Rantes, SDF1-alpha, IFN-gamma, IL-12p40, IL-4 and/or DKK1 is selected as substance of substance group A, with the substances being depleted to more than 50% of the initial concentration of the solution from the biofluid and sequestered in the hydrogel.

11. The method according to one of claims 1 to 10, **characterized in that** in hydrogels of type 2 at least one of the substances FGF-2, TGFb1, EGF, HGF, PLGF, GM-CSF, IL-1 beta, IL-10, IL-6, TNF-alpha and/or sclerostin is selected as substance of substance group B, wherein the substances are bound only up to 50% of the initial concentration of the solution in the hydrogel or released therefrom.

12. The method according to one of claims 1 to 11, **characterized in that** in hydrogels of type 3 at least one of the substances bNGF, PDGF-BB, VEGF-A, eotaxin, GRO-alpha, IP-10, Rantes, SDF1- alpha, IFN-gam ma and/or IL-4 is selected as substance of substance group A, with the substances being depleted to more than 50% of the initial concentration of the solution from the biofluid and sequestered in the hydrogel.

13. The method according to one of claims 1 to 12, **characterized in that** in hydrogels of type 3 at least one of the substances FGF-2, TGFb1, EGF, HGF, PLGF, IL-8, MCP-1, MIP-1 alpha, MIP-1 beta, GM-CSF, IL-1 beta, IL-10, IL-12p40, IL-6, TNF-alpha, sclerostin and/or DKK1 is selected as substance of substance group B, wherein the substances are bound only up to 50% of the initial concentration of the solution in the hydrogel or released therefrom.

14. The method according to one of claims 1 to 13, **characterized in that** in hydrogels of type 4 at least one of the substances bNGF, PDGF-BB, VEGF-A, eotaxin, GRO-alpha, IL-8, IP-10, MCP-1, MIP-1 alpha, MIP-1 beta, Rantes, SDF1-alpha, IFN-gamma, IL-1 beta, IL-10, IL-12p40, IL-4, IL-6, and/or TNF-alpha is selected as substance of substance group A, with the substances being depleted to more than 60% of the initial concentration of the solution from the biofluid and sequestered in the hydrogel.

15. The method according to one of claims 1 to 14, **characterized in that** in hydrogels of type 4 at least one of the substances EGF, PLGF and/or GM-CSF is selected as substance of substance group B, wherein the substances are bound only up to 40% of the initial concentration of the solution in the hydrogel or released therefrom.

16. The method according to one of claims 1 to 15, **characterized in that** hydrogels of type 1, type 2, type 3 or type 4 are precharged with at least one substance of substance group A and/or B in high concentration, whereafter these substances are released from the hydrogel in parallel and independently to change the concentration in the biofluid.

17. The method according to one of claims 1 to 16, **characterized in that** the hydrogels are formed from multiphase materials, i.e. from mixtures of the different types 1-4 of the hydrogel materials or different processing forms (microparticles and bulk hydrogels) or are precharged with different substances or quantities of substances.

18. The method according to one of claims 1 to 17, **characterized in that** the differentiated release of substances from substance group A or B from the sulfated and/or sulfonated hydrogel into the biofluid and the reduced binding of the substances of substance group B in the sulfated and/or sulfonated hydrogel occur simultaneously.

**Revendications**

1. Procédé de séquestration différenciée de substances de différents groupes de substances A et B dans un hydrogel sulfaté et/ou sulfoné et d'appauvrissement des substances du groupe de substances A à partir d'un fluide biologique avec libération différenciée simultanée de substances du groupe de substances A ou B à partir de l'hydrogel sulfaté et/ou sulfoné dans le fluide biologique ou la fixation réduite des substances du groupe de substances B dans l'hydrogel sulfaté et/ou sulfoné, dans lequel l'hydrogel sulfaté et/ou sulfoné est sélectionné parmi un groupe d'hydrogels du type 1, type 2, type 3 ou type 4 et les hydrogels se composent d'éléments constitutifs non chargés et d'éléments constitutifs chargés, lesquels **sont caractérisés en ce que** les éléments constitutifs chargés sont **caractérisés par** un paramètre calculé à partir du nombre de groupes sulfate et/ou sulfonate par unité de répétition divisé par la masse molaire de l'unité de répétition pour le type 1 de 0,0040 à 0,0060 mol/g, pour le type 2 de 0,0025 à 0,0040 mol/g, pour le type 3 de 0,0005 à 0,0025 mol/g et pour le type 4 de 0,0040 à 0,0100 mol/g, et les hydrogels gonflés présentent un module de conservation inférieur à 20 kPa et les propriétés des hydrogels gonflés présentent une concentration en groupes sulfate ou sulfonate en mmol/ml pour le type 1 de 0,09 à 0,20, pour le type 2 de 0,05 à 0,18, pour le type 3 de 0,01 à 0,12 et pour le type 4 de 0,16 à 0,8, et qu'une influence de la concentration de substances du groupe de substances A et de substances du groupe de substances B dans le fluide biologique s'effectue par le choix du type des hydrogels.

2.  Procédé selon la revendication 1, **caractérisé en ce que** les éléments constitutifs chargés des hydrogels présentent un nombre de groupes sulfate et/ou sulfonate par unité de répétition divisé par la masse molaire de l'unité de répétition pour le type 1 de 0,0050 mol/g, pour le type 2 de 0,0035 mol/g ou 0,0038 mol/g, pour le type 3 de 0,0019 mol/g et pour le type 4 de 0,0045 mol/g, et les propriétés des hydrogels gonflés présentent une concentration en sulfate ou sulfonate en mmol/ml pour le type 1 de 0,12 et pour le type 2 de 0,12 ou 0,14 et pour le type 3 de 0,06 et pour le type 4 de 0,28 ou 0,16.

3.  Procédé de séquestration différenciée de substances de différents groupes de substances A et B dans un hydrogel sulfaté et/ou sulfoné et d'appauvrissement des substances du groupe de substances A à partir d'un fluide biologique avec libération différenciée simultanée de substances du groupe de substances B à partir de l'hydrogel sulfaté et/ou sulfoné dans le fluide biologique ou la fixation réduite des substances du groupe de substances B dans l'hydrogel sulfaté et/ou sulfoné, dans lequel l'hydrogel sulfaté et/ou sulfoné est sélectionné parmi un groupe d'hydrogels du type 1, type 2, type 3 ou type 4 et les hydrogels se composent d'éléments constitutifs non chargés et d'éléments constitutifs chargés, lesquels **sont caractérisés en ce que** les éléments constitutifs chargés présentent une concentration en mol de groupes sulfate ou sulfonate par mol de polymère pour le type 1 de 60 à 80, pour le type 2 de 30 à 75, pour le type 3 de 10 à 30 et pour le type 4 de 80 à 120, et les éléments constitutifs chargés dans l'hydrogel présentent une concentration en éléments constitutifs chargés en mmol/ml pour le type 1 de 0,0015 à 0,0025, pour le type 2 de 0,0015 à 0,0030, pour le type 3 de 0,0010 à 0,0040 et pour le type 4 de 0,0018 à 0,0050, et les hydrogels gonflés présentent un module de conservation inférieur à 20 kPa, dans lequel la concentration en les groupes sulfate ou sulfonate présentent en mmol par ml dans l'hydrogel pour le type 1 de 0,09 à 0,20, pour le type 2 de 0,05 à 0,18, pour le type 3 de 0,01 à 0,12 et pour le type 4 de 0,16 à 0,80, et qu'une influence de la concentration de substances du groupe de substances A et de substances du groupe de substances B dans le fluide biologique s'effectue par le choix du type des hydrogels.

4.  Procédé selon la revendication 3, **caractérisé en ce que** les éléments constitutifs chargés présentent une concentration en mol de groupes sulfate ou sulfonate par mol de polymère pour le type 1 de 70,2 et pour le type 2 de 48,4 ou 75,0 et pour le type 3 de 23,4 et pour le type 4 de 90,0, et les éléments constitutifs chargés dans l'hydrogel présentent une concentration en éléments constitutifs chargés en mmol/ml pour le type 1 de 0,0018, pour le type 2 de 0,0025 ou 0,0018, pour le type 3 de 0,0027 et pour le type 4 de 0,0031 ou 0,0018, et les hydrogels gonflés présentent un module de conservation inférieur à 20 kPa, dans lequel la concentration en groupes sulfate ou sulfonate en mmol par ml dans l'hydrogel se monte pour le type 1 à 0,12, pour le type 2 à 0,12 ou 0,14, pour le type 3 à 0,06 et pour le type 4 à 0,28 ou 0,16.

5.  Procédé selon une des revendications 1 à 4, **caractérisé en ce que** la réticulation des éléments constitutifs en l'hydrogel s'effectue par le biais d'une réaction de groupes fonctionnels appropriés à l'élément constitutif chargé et/ou à l'élément constitutif non chargé provenant de la classe des amines, thiols, carboxyles, anhydrides, maléimides, vinylsulfones, acrylates, hydroxyles, isocyanates, époxydes et aldéhydes ou de groupes qui peuvent former des liaisons non covalentes à base de forces électrostatiques ou interactions hydrophobes, liaisons par pont hydrogène ou interactions dipolaires.

6.  Procédé selon une des revendications 1 à 5, **caractérisé en ce que** la réticulation des éléments constitutifs chargés en l'hydrogel s'effectue par le biais d'une réticulation directe au moyen d'une petite molécule de réticulation bifonctionnelle avec une masse molaire d'éléments constitutifs non chargés < 500 g/mol.

7.  Procédé selon une des revendications 1 à 6, **caractérisé en ce que** sont sélectionnés comme éléments constitutifs chargés des glycosaminoglycanes sulfatés obtenus à partir de sources naturelles, comme l'héparine et des héparines désulfatées sélectives, le sulfate de chondroïtine, le sulfate d'héparane, le sulfate de kératane, l'acide hyaluronique sulfaté, ainsi que des glycopolymères sulfatés à base de mannose, lactose, dextrane ainsi que des composés polysulfonés qui portent de l'acide styrènesulfonique (SS), l'acide vinylsufonique (VS), l'acide 2-acrylamido-2-méthylpropanesulfonique (AMPS), l'acide aminopropanesulfonique (APS) ou l'acide anétholesulfonique (AS) comme monomères contenant du soufre et également dans des copolymères avec des unités qui contiennent les groupes de réticulation suscités, et que sont sélectionnés comme éléments constitutifs non chargés des polyéthylèneglycols, poly(2-oxazolines), polyvinylpyrrolidones (PVP), alcools polyvinyliques (PVA) et/ou polyacrylamides (PAM) ou une courte molécule de réticulation bifonctionnelle.

8.  Procédé selon une des revendications 1 à 7, **caractérisé en ce qu'**au moins une des substances bNGF, PDGF-BB, VEGF-A, éotaxine, GRO-alpha, IL-8, IP-10, MCP-1, MIP-1 alpha, MIP-1 bêta, Rantes, SDF1-alpha, IFN-gamma, IL-12p40, IL-4, sclérostine et/ou DKK1 est sélectionnée comme substances du groupe de substances A pour des hydrogels du type 1, dans lequel les substances sont appauvries à plus de 50 % de la concentration initiale de la solution à partir du fluide biologique et séquestrées dans l'hydrogel.

9.  Procédé selon une des revendications 1 à 8, **caractérisé en ce qu'**au moins une des substances FGF-2, TGFb1, EGF, HGF, PLGF, GM-CSF, IL-1 bêta, IL-10, IL-6 et/ou TNF-alpha est sélectionnée comme substances du groupe de substances B pour des hydrogels du type 1, dans lequel les substances sont fixées uniquement jusqu'à 50 % de la concentration initiale de la solution dans l'hydrogel ou libérées de celui-ci.

10. Procédé selon une des revendications 1 à 9, **caractérisé en ce qu'**au moins une des substances bNGF, PDGF-BB, VEGF-A, éotaxine, GRO-alpha, IL-8, IP-10, MCP-1, Rantes, SDF1-alpha, IFN-gamma, IL-12p40, IL-4 et/ou DKK1 est sélectionnée comme substances du groupe de substances A pour des hydrogels du type 2, dans lequel les substances sont appauvries à plus de 50 % de la concentration initiale de la solution à partir du fluide biologique et séquestrées dans l'hydrogel.

11. Procédé selon une des revendications 1 à 10, **caractérisé en ce qu'**au moins une des substances FGF-2, TGFb1, EGF, HGF, PLGF, GM-CSF, IL-1 bêta, IL-10, IL-6, TNF-alpha et/ou sclérostine est sélectionnée comme substances du groupe de substances B pour des hydrogels du type 2, dans lequel les substances sont fixées uniquement jusqu'à 50 % de la concentration initiale de la solution dans l'hydrogel ou libérées de celui-ci.

12. Procédé selon une des revendications 1 à 11, **caractérisé en ce qu'**au moins une des substances bNGF, PDGF-BB, VEGF-A, éotaxine, GRO-alpha, IP-10, Rantes, SDF1-alpha, IFN-gamma et/ou IL-4 est sélectionnée comme substances du groupe de substances A pour des hydrogels du type 3, dans lequel les substances sont appauvries à plus de 50 % de la concentration initiale de la solution à partir du fluide biologique et séquestrées dans l'hydrogel.

13. Procédé selon une des revendications 1 à 12, **caractérisé en ce qu'**au moins une des substances FGF-2, TGFb1, EGF, HGF, PLGF, IL-8, MCP-1, MIP-1 alpha, MIP-1 bêta, GM-CSF, IL-1 bêta, IL-10, IL-12p40, IL-6, TNF-alpha, sclérostine et/ou DKK1 est sélectionnée comme substances du groupe de substances B pour des hydrogels du type 3, dans lequel les substances sont fixées uniquement jusqu'à 50 % de la concentration initiale de la solution dans l'hydrogel ou libérées de celui-ci.

14. Procédé selon une des revendications 1 à 13, **caractérisé en ce qu'**au moins une des substances bNGF, PDGF-BB, VEGF-A, éotaxine, GRO-alpha, IL-8, IP-10, MCP-1, MIP-1 alpha, MIP-1 bêta, Rantes, SDF1-alpha, IFN-gamma, IL-1 bêta, IL-10, IL-12p40, IL-4, IL-6 et/ou TNF-alpha est sélectionnée comme substances du groupe de substances A pour des hydrogels du type 4, dans lequel les substances sont appauvries à plus de 60 % de la concentration initiale de la solution à partir du fluide biologique et séquestrées dans l'hydrogel.

15. Procédé selon une des revendications 1 à 14, **caractérisé en ce qu'**au moins une des substances EGF, PLGF et/ou GM-CSF est sélectionnée comme substances du groupe de substances B pour des hydrogels du type 4, dans lequel les substances sont fixées uniquement jusqu'à 40 % de la concentration initiale de la solution dans l'hydrogel ou libérées de celui-ci.

16. Procédé selon une des revendications 1 à 15, **caractérisé en ce que** des hydrogels du type 1, type 2, type 3 ou type 4 sont préchargés avec au moins une substance du groupe de substances A et/ou B à haute concentration, puis ces substances sont libérées de l'hydrogel en parallèle et indépendamment de la modification de concentration dans le fluide biologique.

17. Procédé selon une des revendications 1 à 16, **caractérisé en ce que** les hydrogels sont réalisés à partir de matériaux à plusieurs phases, c'est-à-dire à partir de mélanges des différents types 1 à 4 des matériaux d'hydrogel ou de différentes formes de traitement (microparticules et hydrogels en vrac) ou sont préchargés avec différentes substances ou quantités de substance.

18. Procédé selon une des revendications 1 à 17, **caractérisé en ce que** la libération différenciée de substances du groupe de substances A ou B de l'hydrogel sulfaté et/ou sulfoné dans le fluide biologique et la fixation réduite des substances du groupe de substances B dans l'hydrogel sulfaté et/ou sulfoné s'effectuent simultanément.

Fig. 1

**EP 3 592 807 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2010060485 A1
- US 5451617 A
- US 5011275 A
- US 2008011412 A
- WO 2014040591 A2

### In der Beschreibung aufgeführte Nicht-Patentliteratur

- Synthetische, Sulfonsäuregruppen enthaltende Polymere, beispielsweise mit Polystyrensulfonsäure (PSS). *J. Phys. Chem. B,* 2007, vol. 111 (13), 3391-3397
- **CAPILA, I. ; LINHARDT, R. J.** *Angew Chem Int Ed Engl,* 2002, vol. 41 (3), 391-412
- **TSURKAN et al.** *Adv. Mater. 2013,* 2013, vol. 25, 2606-2610
- **FREUDENBERG et al.** Journal of Controlled Release. *Journal of Controlled Release : Official Journal of the Controlled Release Society,* 28. Dezember 2015, vol. 220, 79-88
- **UWE FREUDENBERG et al.** Journal of Controlled Release. *Journal of Controlled Release : Official Journal of the Controlled Release Society,* 28. Dezember 2015, vol. 220, 79-88
- **TSURKAN et al.** *Adv. Mater.,* 2013, vol. 25 (18), 2606-2610
- **MICHAEL RUBINSTEIN ; RALPH H. COLBY.** Polymer Physics. Oxford University Press, 2006
- **REFERENZ: GASTEIGER, E. et al.** The Proteomics Protocols Handbook. 2005, 571-607
- **SIGRIST, C. J. A. et al.** *New and continuing developments at ExPASy PROSITE*
- *Nucleic Acids Res.,* 2013, vol. 41
- **SIGRIST, C. J. A et al.** PROSITE: a documented database using patterns and profiles as motif descriptors. *Brief. Bioinform.,* 2002, vol. 3, 265-274